# EUROPÄISCHE PATENTSCHRIFT

(11) **EP 1 633 193 B1**
(45) Veröffentlichungstag und Bekanntmachung des Hinweises auf die Patenterteilung: **04.05.2016**
(21) Anmeldenummer: 04736635.6
(22) Anmeldetag: 11.06.2004
(51) Int. Cl.: A01N 65/00, A01N 31/06, A61K 36/53, A61K 31/045, A61K 8/97, A61Q 5/00, A61Q 11/00, A61Q 19/00, A61Q 19/10, A61P 3/00, A61P 15/00, C14C 9/02, C09J 11/06, C09D 5/34, C09K 3/10, C11D 7/44, C09D 5/14, C04B 24/02, C04B 24/08

(54) **MITTEL GEGEN MIKROORGANISMEN ENTHALTEND PATCHOULIÖL, PATCHOULIALKOHOL UND/ODER DESSEN DERIVATE**
AGENTS AGAINST MICROORGANISMS, CONTAINING PATCHOULI OIL, PATCHOULI ALCOHOL AND/OR THE DERIVATIVES THEREOF
AGENTS DESTINES A LUTTER CONTRE LES MICRO-ORGANISMES, RENFERMANT DE L'HUILE DE PATCHOULI, DE L'ALCOOL DE PATCHOULI ET/OU LEURS DERIVES

(30) Priorität: 17.06.2003 DE 10327138; 17.06.2003 DE 10327134
(43) Veröffentlichungstag der Anmeldung: 15.03.2006
(73) Patentinhaber: Henkel AG & Co. KGaA, 40589 Düsseldorf (DE)
(72) Erfinder: WEIDE, Mirko, 40223 Düsseldorf (DE); SCHLÖSSER, Anja, 41462 Neuss (DE); BOCKMÜHL, Dirk, 42113 Wuppertal (DE); BOLTE, Andreas, 40627 Düsseldorf (DE); BREVES, Roland, 40822 Mettmann (DE)
(86) Internationale Anmeldenummer: PCT/EP2004/006291
(87) Internationale Veröffentlichungsnummer: WO 2004/110153

(56) Entgegenhaltungen:
- WO-A-97/07193
- WO-A-97/30689
- WO-A-97/45511
- WO-A-98/25638
- WO-A-03/018042
- WO-A-2004/054561
- DATABASE CABS [Online] S. ABE ET AL.: "Anticandida albicans activity of essential oils including lemongrass (Cymbopogon citratus) oil and its component, citral" XP002294229 gefunden im CAPLUS accession no. 2004:54523 & S. ABE ET AL.: "Anti-Candida albicans activity of essential oils." NIPPON ISHINKIN GAKKAI ZASSHI, Bd. 44, Nr. 4, 2003, Seiten 285-291,
- I. VALLEJO, L. REBORDINOS, I. G. COLLADO & J. M. CANTORAL FERNANDEZ: "Differential behaviour or mycelial growth of several Botrytis cinerea strains on (...) patchoulol (...) -amended media." J. PHYTOPATHOLOGY, Bd. 149, 2001, Seiten 113-118, XP002294228
- J. A. MORRIS, A. KHETTRY & E. W. SEITZ: "Antimicrobial activity of aroma chemicals and essential oils" J. AM. OIL CHEM. SOC., Bd. 56, 1979, Seiten 595-603, XP000645444
- DEPO YANG ET AL.: "Propriétés antifongiques et antibactériennes, in vitro, de trois huiles essentielles de Patchouli d'origine différentes" ACTA BOT. GALLICA, Bd. 143, Nr. 1, 1996, Seiten 29-35, XP009035749
- DATABASE WPI Section Ch, Week 200129 Derwent Publications Ltd., London, GB; Class B05, AN 2001-275870 XP002294230 & JP 2001 002542 A (LION CORP) 9. Januar 2001 (2001-01-09)
- DATABASE WPI Section Ch, Week 199135 Derwent Publications Ltd., London, GB; Class B04, AN 1991-256638 XP002294231 & JP 03 167132 A (LOTTE CO LTD) 19. Juli 1991 (1991-07-19)
- DATABASE WPI Section Ch, Week 199408 Derwent Publications Ltd., London, GB; Class C05, AN 1994-061962 XP002294232 & JP 06 016517 A (NIPPON KAYAKU KK) 25. Januar 1994 (1994-01-25)

## Beschreibung

Die Erfindung betrifft die Verwendung von Patchouliöl, Patchoulialkohol und/oder dessen Derivaten zur Hemmung der asexuellen Vermehrung von humanpathogenen Pilzen und zur Verminderung der Anhaftung von Mikroorganismen an Oberflächen wie Filtermedien, Klebstoffe, Baustoffe und/oder Bauhilfsstoffe, insbesondere Dichtungsmassen und Klebstoffe, Textilien, Pelze, Papier, Felle oder Leder. Auch betrifft sie diese Verwendung von Patchouliöl, Patchoulialkohol, deren Ester und Ether, Patchoulenol, Norpatchoulenol oder Seychellen in Zubereitungen wie Waschmittel, Reinigungsmittel, Nachspülmittel, Textilbehandlungsmittel, Handwaschmittel, Handgeschirrspülmittel, Maschinengeschirrspülmittel, Baustoffe und Bauhilfsstoffe und Mittel zur Ausrüstung von Baustoffen und/oder Bauhilfsstoffen, Textilien, Pelzen, Papier, Fellen oder Leder.

Die Anhaftung von Mikroorganismen an Oberflächen ist, insbesondere bei pathogenen Mikroorganismen, unerwünscht. Anhaftende Mikroorganismen führen häufig zu Infektionen bzw. zur Reinfektion bei Mensch, Tier und Pflanzen.

Immer häufiger werden empfindliche Textilien, wie z. B. Seide oder Mikrofaser, zu Kleidungsstücken verarbeitet, die nur bei 30 oder 40 °C gewaschen werden56 nicht abgetötet. Insbesondere nach einer Pilzinfektion kann es durch solche auf Kleidungsstücken haftenden, nicht abgetöteten Pilze zu einer Reinfektion kommen.

Weiterhin erkranken Gebissträger häufig an einer oralen Candidose (Soor). An der Oberfläche der Prothese haftende Pilzzellen können bei Kontakt die Schleimhäute besiedeln, die durch Druckstellen oft vorgeschädigt sind.

Um die Reinfektion durch an der Kleidung oder an Kunststoffoberflächen haftenden Mikroorganismen zu verhindern, wurden bisher antimikrobielle Substanzen eingesetzt, die entweder das Wachstum der Mikroorganismen hemmen (Biostatika) oder diese abtöten (Biozide). Nachteilig ist daran, dass solche z. B. in Wasch- und Reinigungsmitteln verwendeten Biozide oder Biostatika die Abwässer belasten und somit auch die mikrobiellen Klärstufen in den Kläranlagen in ihrer Funktion beeinträchtigen. Zudem wird der Selektionsdruck auf die Mikroorganismen zur Resistenzbildung stark erhöht, so dass nach einiger Zeit neue antimikrobielle Substanzen gefunden werden müssen, die gegen diese resistent gewordenen Mikroorganismen wirken.

Pilze, insbesondere Schimmelpilze, treten bspw. im Haushalt an verschiedensten Stellen, bspw. in der Küche oder in feuchten Räumen, wie z.B. im Badezimmer. Schimmelpilze verursachen erhebliche Probleme dadurch, dass die von ihnen in die Raumluft abgegebenen Sporen häufig allergieerzeugend sind. Eine Bekämpfung solcher Pilze mit bioziden Wirkstoffen geht mit einem erhöhten Risiko der Resistenzbildung einher, so dass nach einiger Zeit neue antimikrobielle Substanzen gefunden werden müssen, die gegen diese resistent gewordenen Mikroorganismen wirken. Biozide sind außerdem ökologisch und toxikologisch nicht immer unbedenklich. Zu den unerwünschten Wirkungen der Verbreitung von Schimmelpilzen gehören insbesondere Verfärbungen (beispielsweise an Wänden, Fugendichtungsmassen und anderen Badoberflächen), die durch pigmentierte Sporen hervorgerufen werden.

In den älteren, nicht vorveröffentlichten internationalen Patentanmeldungen PCT/EP02/14306 bzw. PCT/EP02/14322 wird beschrieben, dass Mono-, Sesqui- und/oder Diterpene sowie deren Derivate zur Hemmung der asexuellen Vermehrung von Pilzen bzw. zur Verminderung der Anhaftung von Pilzen verwendet werden können. Als besonders bevorzugter Wirkstoff wird Farnesol genannt.

Vallejo et al. (2001, "Differential behaviour or mycelial growth of several Botrytis cinerea strains on patchoulol-amended media", J. Phytopathology, volume 149, pages 113-118) veröffentlicht die hemmende Wirkung von Patchoulol auf die Myzelbildung eines phytopathogenen Schimmelpilzes.

WO03/018042 und WO98/25638 offenbaren pharmazeutische Zubereitungen enthaltend Patchouli zur Behandlung von viralen Infektionen.

Morris et al. (1979, "Antimicrobial activity of arome chemicals and essential oils", J.AM.OIL. CHEM. SOC, volume 56, pages 595-603) und WO97/45511 offenbaren eine Seife und Waschmittel enthaltend ein Parfüm mit Patchouliöl.

Die antimikrobielle Wirkung von Patchouli in Mund und Zahnpflegemitteln wird in JP2001-002542 und JP 3-167132 offenbart.

Yang et al. (1996, "Propriete antifongue et antibacterienne, in vitro, de trois huiles essentielles de Patchouli d'origine differentes", ACTA BOT. GALLICA, volume 143, no.1, pages 29-35) beschreibt die Verwendung von Patchouliöl in kosmetischen Produkten.

Die Verwendung von Patchouliöl, Patchoulialkohol und/oder Ester und Ether von Patchoulialkohol, Patchoulenol, Norpatchoulenol und Seychellen zur Hemmung der asexuellen Vermehrung von humanpathogenen Pilzen wird im Stand der Technik nicht beschrieben.

Die Aufgabe der Erfindung ist daher, die Nachteile des Standes der Technik zu überwinden und gezielt die negativen Wirkungen sporulierender Pilze zu vermeiden und Mikroorganismen von Oberflächen zu entfernen, ohne einen erhöhten Druck auf die Mikroorganismen zur Resistenzbildung zu erzeugen und ohne diese Oberflächen oder die Abwässer mit bioziden und/oder biostatischen Wirkstoffen bzw. mit Wirkstoffen in bioziden bzw. biostatischen Konzentrationen zu belasten.

Diese Aufgabe wird gelöst durch die Verwendung von Patchouliöl, Patchoulialkohol und/oder Ester und Ether von Patchoulialkohol, Patchoulenol, Norpatchoulenol und Seychellen zur Hemmung der asexuellen Vermehrung von humanpathogenen Pilzen zu nicht medizinischen Zwecken und zur Verminderung der Anhaftung von Mikroorganismen an Oberflächen, wobei die Mikroorganismen ausgewählt sind aus der Gruppe der Bakterien und der humanpathogenen Pilze und wobei es sich bei den Oberflächen um keine Oberflächen am menschlichen oder tierischen Körper handelt.

Unter Mikroorganismen sind Bakterien und humanpathogenen Pilze zu verstehen. Dies schließt bakterielle Endo- oder Exosporen sowie Sporen, die als Fortpflanzungsstrukturen bei Pilzen dienen, mit ein.

Unter Verminderung der Anhaftung ist eine signifikante Reduktion der Zahl der anhaftenden Mikroorganismenzellen zu verstehen. Dabei wird die Anhaftung idealerweise vollständig verhindert. Bevorzugt wird die Anhaftung von Mikroorganismenzellen vermindert oder im Wesentlichen ganz verhindert.

Erfindungsgemäß umfasst der Begriff asexuelle Vermehrung insbesondere Sporenbildung, Knospung und Fragmentierung.

Es wurde nun gefunden, dass die Mikroorganismenzellen in Gegenwart von Patchouliöl, Patchoulialkohol und/oder Ester und Ether von Patchoulialkohol, Patchoulenol, Norpatchoulenol und Seychellen bzw. Lösungen, die diese Stoffe enthalten, kaum bzw. gar nicht an Oberflächen adhärieren.

Überraschenderweise wurde weiterhin gefunden, daß der Einsatz von Patchouliöl, Patchoulialkohol und/oder Ester und Ether von Patchoulialkohol, Patchoulenol, Norpatchoulenol und Seychellen auf oder in durch Pilze befallenen Materialien die asexuelle Vermehrung der Pilze zu unterbinden vermochte, ohne jedoch die Pilze abzutöten.

Gegenstand der vorliegenden Erfindung ist daher die Verwendung von Patchouliöl, Patchoulialkohol und/oder Ester und Ether von Patchoulialkohol, Patchoulenol, Norpatchoulenol und Seychellen zur Hemmung der asexuellen Vermehrung von humanpathogenen Pilzen zu nicht medizinischen Zwecken und zur Verminderung der Anhaftung von Mikroorganismen an Oberflächen wobei die Mikroorganismen ausgewählt sind aus der Gruppe der Bakterien und der humanpathogenen Pilze und wobei es sich bei den Oberflächen um keine Oberflächen am menschlichen oder tierischen Körper handelt.

Patchouliöl wird erfindungsgemäß aus den Pflanzenteilen des Patchoulistrauches (Pogostemon cablin bzw. patchouli sowie P. heyneaus aus der Familie der Laminaceae bzw. Labiatae) gewonnen. Erfindungsgemäß ist es möglich, das Patchouliöl durch Extraktion mit Lösungsmitteln oder deren Gemischen, bevorzugt organischen Lösungsmitteln, insbesondere mit Kohlenwasserstoffen, zu gewinnen (beispielsweise CAS 84238-39-1; CAS 90082-40-9).

Besonders bevorzugt ist mittels Wasserdampfdestillation aus den Blättern gewonnene Patchouliöl (insbesondere CAS 8014-09-3). Bevorzugterweise werden zur Extraktion fermentierte Blätter des Patchoulistrauches eingesetzt. Das Patchouliöl zieht besonders gut auf Oberflächen auf, so dass die Ausrüstung, insbesondere von Textilien aber auch von Kunststoff- und Metalloberflächen, besonders effektiv und einfach durchgeführt werden kann.

Das durch Wasserdampfdestillation der fermentierten Blätter gewonnene Patchouliöl enthält neben Patchoulialkohol, Patchoulenol, Patchoulenon, Norpatchoulenol, Nortetrapatchoulol, Seychellen, α-Patchoulen, β-Patchoulen, α-Guajen sowie α-Bulnesen.

Von Patchoulialkohol können alle Konfigurationsisomere eingesetzt werden, besonders bevorzugt ist jedoch der natürlich vorkommende (-)-Patchoulialkohol.

Unter Derivaten von Patchoulialkohol sind insbesondere neben Estern und Ethern von Patchoulialkohol auch Patchoulenol, Norpatchoulenol und Seychellen zu verstehen.

Vorteilhafterweise werden die Mikroorganismen bei der erfindungsgemäßen Verwendung weder in ihrem Wachstum gehemmt noch abgetötet, es wird lediglich die Adhäsion von Mikroorganismen an Oberflächen und die asexuelle Vermehrung von Pilzen gehemmt bzw. unterdrückt. Der Selektionsdruck für die Bildung von Resistenzen ist daher gering.

Darüber hinaus sind Patchouliöl und Patchoulialkohol toxikologisch, soweit nach heutigem Stand bekannt, unbedenklich, so dass auf die Verwendung für Mensch, Tier und Umwelt wesentlich bedenklicherer biozider Substanzen verzichtet oder zumindest der Einsatz reduziert werden kann. Insbesondere im Vergleich zu anderen Substanzen, die beispielsweise zur Hemmung der asexuellen Vermehrung von Pilzen und/oder zur Verminderung der Adhäsion von Mikroorganismen eingesetzt werden können, wie z.B. Farnesol, ist die Verwendung von Patchouliöl oder Patchoulialkohol bei gleicher Einsatzkonzentration unter dem Gesichtpunkt der toxikologischen Verträglichkeit vorteilhafter.

Ein weiterer Vorteil der Erfindung ist es, dass diese Stoffe im Vergleich mit Bioziden oder Biostatika bereits in geringen Endkonzentrationen wirksam sind und daher kaum Nebenwirkungen befürchtet werden müssen.

Darüber hinaus können die Hemmung der asexuellen Vermehrung und die Verminderung der Anhaftung durch den verringerten Kontakt des menschlichen Körpers mit den Mikroorganismenzellen, beispielsweise der Atemwege mit Schimmelpilzsporen, auch zu einer Verminderung des allergieauslösenden Potentials, insbesondere innerhalb von Wohnräumen, führen.

Gemäß einer besonderen Ausführungsform werden Patchouliöl, Patchoulialkohol und/oder dessen Derivate in solchen Endkonzentrationen eingesetzt, dass sie nicht biozid oder biostatisch, insbesondere fungizid oder fungistatisch, wirken. Ein besonderer Vorteil dieser Ausführungsform ist es, dass das Risiko einer Resistenzbildung gegenüber den verwendeten Stoffen relativ gering ist, da die Mikroorganismen weder abgetötet noch ihr Wachstum gehemmt werden. Die Konzentrationen, bei denen noch keine Hemmung des Wachstums vorliegt, sowie die minimalen Hemmkonzentrationen selbst können in der dem Fachmann bekannter Weise einfach bestimmt werden.

Nur geringe Konzentrationen dieser Wirkstoffe müssen vorhanden sein, damit die asexuelle Vermehrung von Pilzen, insbesondere die Sporulation, gehemmt bzw. die Anhaftung der Mikroorganismen an Oberflächen vermindert bzw. im wesentlichen ganz verhindert wird. Bevorzugt sind die Stoffe zu 0,00001 bis 1 Gew.-% und insbesondere zu 0,0001 bis 0,5 Gew.-% enthalten. Besonders bevorzugt sind Bereiche zwischen 0,0001 und 0,1 Gew.-%.

Besonders bevorzugt sind bei der Verwendung zur Hemmung der asexuellen Vermehrung Konzentrationsbereiche zwischen 0,0001 und 0,05 Gew.-% geeignet. Speziell bei Patchoulialkohol sind Mengen zwischen 0,0001 und 1,0 Gew.-% besonders bevorzugt.

Die Konzentrationen, die im Endprodukt zum gewünschten Ergebnis führen, sind bedeutend geringer als die angegebenen, da für viele Produkte Verdünnungen berücksichtigt werden müssen. Für Waschmittel muss beispielsweise mit einem Verdünnungsfaktor (Verhältnis Waschmittelkonzentrat : Wasser) von 1:20 bis zu 1:200 gerechnet werden. Häufig liegt das Verdünnungsverhältnis für Waschmittel zwischen 1:60 und 1:100, beispielsweise 1:80.
In der fertigen Anwendungslösung zeigen insbesondere Konzentrationen von 0,0001 bis 1 Gew.-% eine besonders gute Wirkung (insbesondere bzgl. der Sporulationshemmung). Bevorzugt werden 0,001 bis 0,1 Gew.-%, beispielsweise 0,01 Gew.-%, eingesetzt.
Für Patchoulialkohol wären beispielsweise Konzentrationen von 0,001 bis 1,5 Gew.-% , insbesondere von 0,01 bis 0,8 Gew.-% geeignet.

Für den Einsatz von Patchouliöl, insbesondere für die Verwendung zur Hemmung der asexuellen Vermehrung von Pilzen, wären beispielsweise Konzentrationen von 0,001 bis 1,0 Gew.-%, insbesondere von 0,01 bis 0,5 Gew.-% geeignet.

Unter Mikroorganismen sind Bakterien und humanpathogene Pilze zu verstehen. Besonders bevorzugt sind dabei von den Pilzen die Hefen, Schimmelpilze und die keratinophilen Pilze.

Die erfindungsgemäß verwendbaren Wirkstoffe Patchouliöl, Patchoulialkohol und/oder Ester und Ether von Patchoulialkohol, Patchoulenol, Norpatchoulenol und Seychellen sind für den Einsatz (insbesondere zur Hemmung der asexuellen Vermehrung von Pilzen) insbesondere gegen solche Pilze geeignet, die in den Stammlisten "DSMZ - List of Filamentous Fungi" und "DSMZ - List of Yeasts" der DSMZ (Deutsche Stammsammlung von Mikroorganismen und Zellkulturen GmbH, Braunschweig) aufgeführt sind. Die Listen sind im Internet unter der Adresse (http://www.dsmz.de/species/fungi.htm) bzw. (http://www.dsmz.de/species/yeasts.htm) einsehbar.

Unter Schimmelpilzen sind gemäß der vorliegenden Erfindung solche Pilze zu verstehen, die ihren Lebensraum im Boden, auf Lebens- und/oder Futtermitteln oder in konzentrierten Nährlösungen haben, ein typisches Mycel bilden und ihre Nährstoffe aus organischen Substanzen gewinnen, die sie dadurch zersetzen (saprobiontische bzw. saprophytische Lebensweise). Des weiteren vermehren sie sich überwiegend ungeschlechtlich durch Sporen (insbesondere Sporangiosporen oder Konidien) und bilden, wenn überhaupt, nur sehr kleine sexuelle Fortpflanzungsorgane aus.

Dazu sind zum Beispiel Spezies aus den Klassen Ascomycota, Basidiomycota, Deuteromycota und Zygomycota zu zählen, insbesondere alle Spezies der Gattungen Aspergillus, Penicillium, Cladosporium und Mucor sowie *Stachybotrys, Phoma, Alternaria, Aureobasidium, Ulocladium, Epicoccum, Stemphyllium, Paecilomyces, Trichoderma, Scopulariopsis, Wallemia, Botrytis, Verticillium und Chaetonium.*

Zu den Ascomycota gehören hier insbesondere alle Spezies der Gattungen Aspergillus, Penicillium und Cladosporium. Diese Pilze bilden Sporen aus, die bei Kontakt mit der Haut oder den Atemwegen ein stark allergieauslösendes Potential aufweisen. Zu den Basidiomycota ist beispielsweise Cryptococcus neoformans zu zählen. Zu den Deuteromycota sind alle als Schimmelpilze bekannten Gattungen zu zählen, insbesondere solche, die durch das Fehlen eines sexuellen Stadiums nicht den Klassen Ascomycota, Basidiomycota oder Zygomycota zugeordnet werden.

Patchouliöl, Patchoulialkohol und/oder dessen Derivate sind besonders bevorzugt zur Hemmung der asexuellen Vermehrung, insbesondere der Sporulation, aller Species der Gattung Aspergillus, und der Verminderung deren Anhaftung an Oberflächen geeignet, ganz besonders bevorzugt bei Species, die ausgewählt sind unter Aspergillus aculeatus, Aspergillus albus, Aspergillus alliaceus, Aspergillus asperescens, Aspergillus awamori, Aspergillus candidus, Aspergillus carbonarius, Aspergillus carneus, Aspergillus chevalieri, Aspergillus chevalieri var. intermedius, Aspergillus clavatus, Aspergillus ficuum, Aspergillus flavipes, Aspergillus flavus, Aspergillus foetidus, Aspergillus fumigatus, Aspergillus giganteus, Aspergillus humicola, Aspergillus intermedius, Aspergillus japonicus, Aspergillus nidulans, Aspergillus niger, Aspergillus niveus, Aspergillus ochraceus, Aspergillus oryzae, Aspergillus ostianus, Aspergillus parasiticus, Aspergillus parasiticus var. globosus, Aspergillus penicillioides, Aspergillus phoenicis, Aspergillus rugulosus, Aspergillus sclerotiorum, Aspergillus sojae var. gymnosardae, Aspergillus sydowi, Aspergillus tamarii, Aspergillus terreus, Aspergillus terricola, Aspergillus toxicarius, Aspergillus unguis, Aspergillus ustus, Aspergillus versicolor, Aspergillus vitricolae und Aspergillus wentii. Besonders bevorzugt wird die Sporulation von Aspergillus flavus und Apsergillus nidulans gehemmt sowie deren Anhaftung vermindert bzw. im wesentlichen ganz verhindert.

Gemäß einer besonders bevorzugten Ausführungsform werden Patchouliöl, Patchoulialkohol und/oder Ester und Ether von Patchoulialkohol, Patchoulenol, Norpatchoulenol und Seychellen ganz besonders bevorzugt zur Hemmung der Sporenbildung bei Spezies der Gattung Aspergillus eingesetzt, die ausgewählt sind unter *Aspergillus flavus* und *Aspergillus nidulans.*

Gemäß einer bevorzugten Ausführungsform der vorliegenden Erfindung werden die Patchouliöl, Patchoulialkohol und/oder Ester und Ether von Patchoulialkohol, Patchoulenol, Norpatchoulenol und Seychellen zur Hemmung der Sporen bildung verwendet. Unter Sporenbildung ist dabei sowohl die Bildung von Vermehrungsformen z.B. Konidien, Gonitocysten, Sporangiosporen, Arthrosporen, Blastosporen und deren assoziierten Organen (z.B. Konidiophoren) als auch von Dauerformen (z.B. Chlamydosporen) zu verstehen.

Da Pilzsporen, insbesondere Schimmelpilzsporen, in der Raumluft ubiquitär vorhanden sind, kann ein Pilzbefall nicht grundsätzlich verhindert werden. Die Hemmung der Sporulation der auswachsenden Pilzkolonien sowie die Verminderung deren Anhaftung an Oberflächen bietet allerdings die Möglichkeit, das Risiko einer Allergie, insbesondere einer Schimmelpilzallergie, erheblich zu verringern und die Verbreitung des Pilzes vollständig aufzuhalten bzw. beträchtlich zu verzögern. Verfärbungen aufgrund von Sporenbildung werden ebenfalls stark vermindert oder vollständig verhindert.

Die Verwendung von Patchouliöl, Patchoulialkohol und/oder Ester und Ether von Patchoulialkohol, Patchoulenol, Norpatchoulenol und Seychellen zur Hemmung der Sporenbildung weist weiterhin den Vorteil auf, dass die zur Sporulationshemmung einzusetzende notwendige Konzentration überraschenderweise im Vergleich zu anderen Sesquiterpenen, beispielsweise Farnesol, noch deutlich niedriger ist. So kann ein vergleichbarer Effekt auch mit geringerer Konzentration an Wirkstoff erreicht werden.

Zudem weisen Patchouliöl und Patchoulialkohol einen holzigen Duft auf, der den entsprechenden erfindungsgemäßen Stoffen eine angenehme Duftnote verleiht und ggf. weiteren Parfümzusatz überflüssig machen kann.

Hefen im erfindungsgemäßen Zusammenhang sind einzellige Pilze, die sich überwiegend durch Sprossung vermehren. Hefepilze stellen keine eigenständige taxonomische Kategorie im System der Pilze dar. Systematisch gehören die meisten der Hefen zu den Endomycetes. Daneben treten aber auch bei verschiedenen anderen Pilzen im Entwicklungszyklus oder unter bestimmten Umweltbedingungen Sprosszellstadien auf, die als Hefestadien bezeichnet werden. Solche einzellig, hefeartig sprossende Wuchsformen treten bei den Ascomyceten, aber auch bei den Zygomyceten, Basidomyceten und Deuteromyceten auf. Erfindungsgemäß sind auch alle diese Wuchsformen unter Hefen zu verstehen.

Nach einer weiteren besonderen Ausführungsform wird durch die Verwendung von Patchouliöl, Patchoulialkohol und/oder Ester und Ether von Patchoulialkohol, Patchoulenol, Norpatchoulenol und Seychellen die Anhaftung von humanpathogenen Pilzen vermindert und/oder deren asexuelle Vermehrung gehemmt. Dazu sind zum Beispiel die humanpathogenen Spezies aus den Klassen Ascomycota, Basidiomycota, Deuteromycota und Zygomycota zu zählen, insbesondere die humanpathogenen Formen von Candida.

Die humanpathogenen Candida Spezies besiedeln auch bei gesunden Menschen Haut und Schleimhäute. Bei starker Vermehrung der Pilzzellen, z. B. nach Schädigung der bakteriellen Schleimhautflora durch Antibiotika, verursachen sie aber lokale Entzündungen, die auch als Soor bezeichnet werden. Insbesondere treten diese im Mund- und Genitalbereich auf (sogenannter Mund- bzw. Vaginal-Soor). Bekannt sind auch Haut- und Windelsoor. Die Schleimhaut ist gerötet, es zeigen sich Läsionen, ein weißer Belag und Juckreiz treten auf.

Nach einer weiteren besonders bevorzugten Ausführungsform wird die Anhaftung von Pilzen der Spezies der Candida vermindert (im weiteren mit C. abgekürzt), wie z. B.: *C. aaseri, C. actiscondensi, C. acutus, C. agrestis, C. albicans, C. amapae, C. anatomiae, C. ancudensis, C. antarctica, C. antillancae, C. apicola, C. apis, C. aquaetextoris, C. aquatica, C. atlantica, C. atmosphaerica, C. auringiensis, C. azyma, C. beechii, C. benhamii, C. bertae, C. berthetii, C. blankii, C. boidinii, C. boleticola, C. bombi, C. bondarzewiae, C. brumptii, C. buffonii, C. buinensis, C. cacaoi, C. cantarellii, C. capsuligena, C. cariosilignicola, C. caseinolytica, C. castellii, C. catenulata, C. chalmersi, C. chilensis, C. chiropterorum, C. ciferii, C. claussenii, C. coipomensis, C. colliculosa, C. conglobata, C. curiosa, C. cylindracea, C. dendrica, C. dendronema, C. deserticola, C. diddensiae, C. diffluens, C. diversa, C. drymisii, C. dubliniensis, C. edax, C. entomophila, C. eremophila, C. ergatensis, C. ernobii*, *C. etchellsii, C. etchellsii, C. ethanolica, C. ethanothermophilum, C. evantina, C. fabianii, C. famata, C. fennica, C. flareri, C. fluviotilis, C. fragariorum, C. fragi, C. fragicola, C. freyschussii, C. friedrichii, C. fructus, C. fusiformata, C. geochares, C. glabrata, C. glaebosa, C. graminis, C. gropengiesseri, C. guilliermondii, C. haemulonii, C. hellenica, C. heveanensis, C. holmii, C. homilentoma, C. humicola, C. humilis, C. iberica, C. incommunis, C. inconspicua, C. ingens, C. insectalens, C. insectamans, C. insectorum, C. intermedia, C ishiwadae, C. japonica, C. javanica, C. karawaiewii, C. kefyr, C. kruisii, C. krusei, C. krusoides, C. lactiscondensi, C. lambica, C. laureliae, C. lipolytica, C lianquihuensis, C. lodderae, C. lusitaniae, C. magnoliae, C. malicola, C. maltosa, C. maris, C. maritima, C. melibiosica, C. melinii, C. membranaefaciens, C. mesenterica, C. methanosorbosa, C milleri, C. mogii, C. molischiana, C. monosa, C. montana, C. mucilaginosa, C. multis-gemmis, C. musae, C. muscorum, C. mycoderma, C. naeodendra, C. nakasei, C. nemodendra, C. nitratophila, C. norvegensis, C novakii, C. oleophila, C. oregonensis, C. palmyrana, C. paludigena, C. parapsilosis, C. pararugosa, C. pelliculosa, C. peltata, C. periphelosum, C. petrohuensis, C. pignaliae, C. pintolopesii, C. pinus, C. placentae, C. polymorpha, C. populi, C. pseudotropicalis, C. psychrophila, C. pulcherrima, C. punica, C. quercitrusa, C. quercuum, C. railenensis, C. ralunensis, C. reukaufii, C. rhagii, C. rugopelliculosa, C. rugosa, C. saitoana, C. sake, C. saimanticensis, C. santamariae, C. santjacobensis, C. savonica, C. schatavii, C. sequanensis, C. shehatae, C. silvae, C. silvanorum, C. silvicultrix, C. solani, C. sonorensis, C. sophiae-reginae, C. sorboxylosa, C. spandovensis, C. sphaerica, C. stellata, C. stellatoidea, C. succiphila, C. sydowiorum, C. tanzawaensis, C. tenuis, C. tepae, C. terebra, C. torresii, C. tropicalis, C. tsuchiyae, C. tsukubaensis, C. utilis, C. valdiviana, C. valida, C. vanderwaltii, C. vartiovaarai, C. versatilis, C. vini, C. viswanathii, C. wickerhamii, C. xestobii, C. zeylanoides.*

Nach einer weiteren bevorzugten Ausführungsform wird die Anhaftung von Pilzen der Spezies *Rhodotorula spp., Cryptococcus spp., Exophilia spp. , Hormoconis spp.* vermindert.

Insbesondere wird durch die erfindungsgemäße Verwendung bevorzugt die Anhaftung der medizinisch relevanten Formen von Candida vermindert, beispielsweise von: *C. albicans, C. boidinii, C. catenulata, C. ciferii, C. dubliniensis, C. glabrata, C. guilliermondii, C. haemulonii, C. kefyr, C. krusei, C. lipolytica, C. lusitaniae, C. norvegensis, C. parapsilosis, C. pulcherrima, C. rugosa, C. tropicalis, C. utilis, C. viswanathii.* Insbesondere bevorzugt sind *C. albicans, C. stellatoidea, C. tropicalis, C. glabrata* und *C. parapsilosis.* Die Mycel-Form von Candida wird als humanpathogene Form des Pilzes betrachtet. Eine Verminderung der Anhaftung von Candida beispielsweise an Textilien oder Kunststoffen vermindert das Risiko der Reinfektion, ohne die Bildung von Resistenzen zu erhöhen.

Unter keratinophilen Pilzen sind solche Haut- und/oder Haarpilze zu verstehen, die in verhornter Haut und deren Anhangsgebilden (insbesondere Haaren und/oder Nägeln) wachsen. Insbesondere sind darunter Dermatophyten und alle Spezies der Gattung Malassezia zu verstehen. Unter Dermatophyten sind erfindungsgemäß insbesondere alle Spezies der Gattungen Trichophyton, Microsporum und Epidermophyton zu verstehen.

Der keratinophile Pilz Malassezia, ein Hefepilz, gilt als Erreger von vermehrter Schuppenbildung der Haut, beispielsweise auf dem Kopf (Haarschuppen). Außerdem wird dieser Organismus als Auslöser der Hautkrankheit Pityriasis versicolor betrachtet. Insbesondere ist es daher von Vorteil, die Adhäsion von Malassezia, insbesondere die Spezies *M. furfur (auch bekannt unter dem Namen Pityrosporum ovale), M. pachydermatis, M. sympodialis* und/oder M. *globosa* zu vermindern bzw. im wesentlichen zu verhindern.

Gemäß einer bevorzugten Ausführungsform sind die keratinophilen Pilze ausgewählt aus *Trichophyton mentagrophytes, T. rubrum, T. asteroides, T. concentrium, T. equinum, T. meginii, T. gallinae, T. tonsurans, T. schoenleinii, T. terrestre, T. verrucosum, T. violaceum, Microsporum canis, Microsporum audounii, M. gypseum, Epidermophyton flossocum, Malassezia furfur, M. sympodialis, M. globosa und M. pachydermatis.*

Nach einer weiteren bevorzugten Ausführungsform wird durch die Verwendung von Patchouliöl, Patchoulialkohol und/oder Ester und Ether von Patchoulialkohol, Patchoulenol, Norpatchoulenol und Seychellen die Anhaftung von Dermatophyten an Oberflächen vermindert. Insbesondere sind die Dermatophyten ausgewählt aus *Trichophyton mentagrophytes, T. rubrum, T. asteroides, T. concentrium, T. equinum, T. meginii, T. gallinae, T. tonsurans, T. schoenleinii, T. terrestre, T. verrucosum, T. violaceum, Microsporum canis, Microsporum audounii, M. gypseum und Epidermophyton flossocum.*

Gemäß einer besonders bevorzugten Ausführungsform wird durch die Verwendung von Patchouliöl, Patchoulialkohol und/oder Ester und Ether von Patchoulialkohol, Patchoulenol, Norpatchoulenol und Seychellen die Anhaftung von Bakterien vermindert, wie z.B. die folgenden gramnegativen und grampositiven Bakterien, insbesondere die pathogenen Bakterien *Propionibacterium acnes, Stapylococcus aureus,* Streptokokken Gruppe A (beta-hämolysierende S.), *S. pyogenes, Corynebacterium* spp. (insbesondere *C. tenuis, C. diphtheriae, C. minutissimum*), *Micrococcus spp.* (insbesondere *M. sedentarius*), *Bacillus anthracis, Neisseria meningitidis, N. gonorrhoeae, Pseudomonas aeruginosa, P. pseudomallei, Borrelia burgdorferi, Treponema pallidum, Mycobacterium tuberculosis, Mycobacterium spp., Escherichia coli* sowie *Streptococcus* spec. (insbesondere *S. gordonii, S. mutans*)*, Actinomyces* spec. (insbesondere A. *naeslundii*), *Salmonella* spec., *Actinobacteria* (insbesondere *Brachybacterium* spec.), *alpha-Proteobacteria* (insbesondere *Agrobacterium* spec.), *beta-Proteobacteria* (insbesondere *Nitrosomonas* spec.), *Aquabacterium* spec., *Hydrogenophaga, gamma-Proteobacteria* (insbesondere) *Stenotrophomonas* spec., *Xanthomonas* spec (campestris), *Neisseria* spec., *Haemophilus* spec. sowie alle Mikroorganismen, die gem. "Paster et al. J. Bac. 183, 12, 2001, 3770-3783" beschrieben wurden.

Algen sind ein- bis vielzellige, verschieden gefärbte, primär photoautotrophe Pflanzen bzw. photoautotrophe Bakterien von meist thallophytischer Organisation, deren Gameten und Sporen bildende Organe in der Regel einzellig sind und eventuell Hüllen aus sterilen Zellen besitzen. Algen werden nach ihrer Pigmentzusammensetzung in Grün-, Rot-, Blau- und Braunalgen unterschieden, wobei vor allem Grün- und Blaualgen an Fassaden und Baustoffen relevant sind. Die relevanten Vertreter der Blaualgen- (Cyanobacteria) sind aus den Gattungen Anabaena, Anacystis, insb. Anacystis montana, Gloeocapsa, Lyngbia, Nostoc, Oscillatoria, insb. Oscillatoria lutea, Phormidium, Schiszothrix und Scytonema sowie die Grünalgen- (Chlorophyta) Gattungen Chlorella, Choricystis, Chlamydomonas, Chlorococcum, Stichcoccus, insb. Stichcoccus bacillaris, Ulothrix und Trentepholia, insb. Trentepholia odorata. Erfindungsgemäß kann nun die Anhaftung von Algen auf Oberflächen insbesondere in sehr feuchten Räumen sowie Aquarien, aber auch auf solchen Oberflächen, die der Witterung ausgesetzt sind, wie z.B. Baustoffen, darunter insbesondere Dichtstoffe bzw. Versiegelungen, durch die Verwendung von Patchchouliöl, Patchoulialkohol und/oder dessen Derivaten verhindert werden.

Nach einer weiteren besonders bevorzugten Ausführungsform wird die Anhaftung von Mikroorganismen auf den Oberflächen, die häufig in Kontakt mit dem menschlichen Körper kommen, vermindert bzw. im Wesentlichen völlig verhindert. Dabei sind insbesondere abiotische, technische (bzw. technisch hergestellte) Oberflächen gemeint. Im Sinne dieser besonderen Ausführungsform ist daher menschliches Gewebe nicht darunter zu verstehen.

Bei nicht ausreichender Reinigung dieser Oberflächen kann es durch die Anhaftung von Mikroorganismen zur Reinfektion bereits befallener Körperstellen bzw. zu weiteren Neuinfektionen kommen.

Gemäß einer ganz besonders bevorzugten Ausführungsform wird die asexuelle Vermehrung und/oder die Anhaftung von Mikroorganismen an solchen Oberflächen wie Textilien, Keramiken, Metallen und/oder Kunststoffen oder in oder auf Filtermedien, Baustoffen, Bauhilfsstoffen, Pelzen, Papier, Fellen, Leder gehemmt bzw. vermindert.

Insbesondere handelt es sich dabei um Wäsche, Sanitäreinrichtungen, Bodenbeläge, Schuhe, Leder, aus Gummi hergestellte Gebrauchsgegenstände, Prothesen oder Zahnersatz. Pilzinfektionen von Schleimhäuten, insbesondere im Mund- und Genitalbereich, können einfach und erfolgreich durch Antimykotika sowie bakterielle Infektionen durch Antibiotika therapiert werden. Dabei ist es aber sehr wichtig, dass die mit den Mikroorganismenzellen kontaminierten Oberflächen, beispielsweise bei Pilzzellen Wäsche, insbesondere Unterwäsche oder Strümpfe, von diesen gereinigt werden. Im Falle empfindlicher Textilien, wie z. B. Seide oder Mikrofaser sowie Synthetikgewebe, kann dies nicht durch eine erhöhte Waschtemperatur erfolgen, ohne dass das Material geschädigt wird. Auch die Verwendung von stark bleichehaltigen Vollwaschmitteln ist aufgrund einer möglichen Materialschädigung nicht zu empfehlen.

Die Verminderung der Anhaftung bzw. der asexuellen Vermehrung an Textilien oder Kunststoffoberflächen verhindert sehr häufig eine Reinfektion der bereits befallenen Körperbereiche. Die Verminderung der Anhaftung von Mikroorganismen an Keramiken, Kunststoffen oder Metallen, insbesondere an Prothesen oder Zahnersatz, verringert das Infektions- bzw. Reinfektionsrisiko, ohne die Haut, die Schleimhäute oder die Abwässer mit biozid oder biostatisch bzw. virostatisch wirkenden Substanzen zu belasten. Ebenso können Katheter sowie andere aus Kunststoff oder Metallen hergestellte medizinische Geräte und/oder Prothesen durch die Verwendung solcher Stoffe beispielsweise in Spülungen oder Reinigungsmitteln von der Anhaftung befreit werden.

Weiterer Gegenstande der vorliegenden Erfindung ist die Verwendung von Patchouliöl, Patchoulialkohol und/oder Ester und Ether von Patchoulialkohol, Patchoulenol, Norpatchoulenol und Seychellen in Waschmitteln, Reinigungsmitteln, Nachspülmitteln, Handwaschmitteln, Handgeschirrspülmitteln, Maschinengeschirrspülmitteln und in Mitteln zur Ausrüstung von Oberflächen und/oder Verpackungen, insbesondere solchen, die mit Lebensmitteln in Berührung kommen, Filtermedien, Baustoffen, Bauhilfsstoffen, Textilien, Pelzen, Papier, Fellen oder Leder, zur Hemmung der asexuellen Vermehrung von Pilzen sowie zur Verminderung der Anhaftung von Mikroorganismen an Oberflächen.

Nach einer besonderen Ausführungsform werden Patchouliöl, Patchoulialkohol und/oder dessen Derivate zu Wasch- und/oder Reinigungsmitteln oder zur Mundpflege- bzw. Gebissreinigungsprodukten zugegeben. Insbesondere die modernen Textilfasern, die nicht mit Vollwaschmittel bzw. bei hohen Temperaturen gewaschen werden können, können durch übliche Feinwaschmittel bzw. Waschtemperaturen bei 30 oder 40 °C nicht vollständig von Mikroorganismenanhaftungen befreit werden. Ein Vorteil des Einsatzes solcher Zusatzstoffe in Wasch- und Reinigungsmitteln ist es, dass trotz geringer Abwasserbelastung sowie geringem Risiko zur Resistenzenbildung Kleidungsstücke von der Anhaftung von Mikroorganismen befreit werden können. Zahnersatz, insbesondere Gebisse, können durch die Verwendung solcher Stoffe in Mund-, Zahn- und/oder Zahnprothesenpflegeprodukten wirksam, einfach und ohne Belastung der behandelten Oberfläche mit stark biozid wirkenden, möglicherweise bedingt toxischen Substanzen von der Mikroorganismenanhaftung gereinigt werden. Insbesondere eignen sich für die Mund-, Zahn- und/oder Zahnprothesenpflege Patchouliöl, Patchoulialkohol und/oder dessen Derivate.

Ein weiterer Gegenstand der Erfindung ist die erfindungsgemäße Verwendung durch Wasch- und/oder Reinigungsmittel, enthaltend 0,000001 bis 3 Gew.-% an Patchouliöl, Patchoulialkohol und/oder Ester und Ether von Patchoulialkohol, Patchoulenol, Norpatchoulenol und Seychellen. Besonders bevorzugt sind Konzentrationen von 0,00001 bis 1,0 Gew.-% und insbesondere 0,0001 bis 0,5 Gew.-%. Ganz besonders bevorzugt enthalten die Wasch- und Reinigungsmittel 0,0001 bis 0,05 Gew.-%, insbesondere bis 0,01 Gew.-%, dieser Stoffe.
Solche Wasch- und Reinigungsmittel können ohne Belastung der Abwässer relativ geringe Mengen der Substanzen enthalten. Da sie in konzentrierter Form eingesetzt und auf die entsprechend wirksamen Konzentrationen in der Waschlauge verdünnt werden, müssen die Wirkstoffe in entsprechend höherer Konzentration eingesetzt werden. Üblich sind Verdünnungen der Wasch- und Reinigungsmittel mit Wasser zwischen 1:40 und 1:200.
Solche Stoffe können erfindungsgemäß auch zu Reinigungsmitteln, die zum Säubern harter Oberflächen, wie zum Beispiel von Böden, Kacheln, Fliesen, Kunststoffen sowie anderen harten Oberflächen im Haushalt, insbesondere in feuchten Räumen (z.B. Badezimmer), in öffentlichen sanitären Anlagen, in Schwimmbädern, Saunen, Sportanlagen oder in Arzt- oder Massagepraxen zugegeben werden. Sie können dort vorteilhafterweise die unerwünschte Verfärbung von Oberflächen durch Bildung gefärbter Sporen (z.B. schwarz von Aspergillus niger) verhindern. Auch Duschvorhänge und andere Badetextilien sowie Kunststoffe können vor Schimmel(-sporen)-bedingten Verfärbungen geschützt werden.
Die Hemmung der asexuellen Vermehrung von Pilzen auf Textilien oder Kunststoffoberflächen verhindert häufig eine Reinfektion von zuvor bereits befallenen Körperbereichen. Die Hemmung der asexuellen Vermehrung von Pilzen auf Keramiken, Kunststoffen oder Metallen verringert das Infektions- bzw. Reinfektionsrisiko, ohne die Haut, die Schleimhäute oder die Abwässer mit fungizid bzw. fungistatisch wirkenden Substanzen zu belasten. Ebenso können Katheter sowie andere aus Kunststoff oder Metallen hergestellte medizinische Geräte und/oder Prothesen durch die Verwendung von Patchouliöl, Patchoulialkohol und/oder Ester und Ether von Patchoulialkohol, Patchoulenol, Norpatchoulenol und Seychellen beispielsweise in Spülungen oder Reinigungsmitteln weitgehend von Pilzen freigehalten werden.

Des weiteren wird durch solche Reinigungsmittel die Anhaftung von Mikroorganismen, die in solchen Bereichen aufgrund günstiger Bedingungen besonders häufig auftreten, vermindert bzw. ganz verhindert. Dies hat den Vorteil, dass eine Infektion bzw. Reinfektion mit solchen, insbesondere humanpathogenen Mikroorganismen, erschwert und im besten Fall ganz verhindert wird.

Neben den pathogenen Mikroorganismen (insbesondere Pilze und Bakterien) sind auf solchen Oberflächen insbesondere Pseudomonas aeruginosa, Salmonelle spec., Actinobacteria (insbesondere Brachybacterium spec.), alpha-Proteobacteria (insbesondere Agrobacterium spec.), beta-Proteobacteria (insbesondere Ntrosomonas spec., Aquabacterium spec., Hydrogenophaga), gamma-Proteobacteria (insbesondere Stenotrophomonas spec., Xanthomonas spec (campestris)).

Unter Wasch- und Reinigungsmitteln werden im erfindungsgemäßen Zusammenhang im weitesten Sinn tensidhaltige Zubereitungen in fester Form (Partikel, Pulver usw.), halbfester Form (Pasten usw.), flüssiger Form (Lösungen, Emulsionen, Suspensionen, Gele usw.) und gasähnlicher Form (Aerosole usw.) verstanden, die im Hinblick auf eine vorteilhafte Wirkung bei der Anwendung ein Tensid oder mehrere Tenside enthalten, üblicherweise neben weiteren Komponenten, die für den jeweiligen Anwendungszweck üblich sind. Beispiele für solche tensidhaltige Zubereitungen sind tensidhaltige Waschmittelzubereitungen, tensidhaltige Reinigungsmittel für harte Oberflächen, oder tensidhaltige Aviviermittelzubereitungen, die jeweils fest oder flüssig sein können, jedoch auch in einer Form vorliegen können, die feste und flüssige Komponenten oder Teilmengen der Komponenten nebeneinander umfasst.

Die Wasch- und Reinigungsmittel können üblicherweise enthaltende Inhaltsstoffe enthalten, wie anionische, nichtionische, kationische und amphotere Tenside, anorganische und organische Buildersubstanzen, spezielle Polymere (beispielsweise solche mit Cobuildereigenschaften), Schauminhibitoren, Farbstoffe und ggf. zusätzliche Duftstoffe (Parfums), Bleichmittel (wie beispielsweise Peroxo-Bleichmittel und Chlor-Bleichmittel), Bleichaktivatoren, Bleichstabilisatoren, Bleichkatalysatoren, Enzyme und Vergrauungsinhibitoren, ohne dass die Inhaltsstoffe auf diese Substanzgruppen beschränkt sind. Häufig sind wichtige Inhaltsstoffe dieser Zubereitungen auch Waschhilfsmittel, für die beispielhaft und nicht beschränkend optische Aufheller, UV-Schutzsubstanzen, sog. Soil Repellents, also Polymere, die einer Wiederanschmutzung von Fasern entgegenwirken, verstanden werden. Die einzelnen Substanzgruppen werden im weiteren näher erläutert.

Für den Fall, dass die Zubereitungen zumindest zum Teil als Formkörper vorliegen, können auch Binde- und Desintegrationshilfsmittel enthalten sein.

Als Tenside können anionische, nichtionische, zwitterionische und kationische Tenside eingesetzt werden.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, in Betracht, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von 2-Sulfofettsäuren (Estersulfonate), z.B. die 2-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. In Wasch- und Reinigungsmitteln sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate, welche beispielsweise gemäß den US-Patentschriften 3,234,258 oder 5,075,041 hergestellt werden und als Handelsprodukte der Shell Oil Company unter dem Namen DAN^{®} erhalten werden können, sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Wasch- und Reinigungsmitteln aufgrund ihres hohen Schaumverhaltens nur in relativ geringen Mengen, beispielsweise in Mengen von 1 bis 5 Gew.-%, eingesetzt.

Weitere geeignete Aniontenside sind auch die Salze der Alkylsulfobernsteinsäure, die auch als Sulfosuccinate oder als Sulfobernsteinsäureester bezeichnet werden, und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.
Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische.
Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Bevorzugt sind die Natrium- oder Kaliumsalze, insbesondere die Natriumsalze. Die Tenside können ebenfalls in Form ihrer Magnesiumsalze eingesetzt werden.

Im Rahmen der vorliegenden erfindungsgemäßen Verwendung sind solche Mittel bevorzugt, die 5 bis 50 Gew.-%, vorzugsweise 7,5 bis 40 Gew.-% und insbesondere 15 bis 25 Gew.-% eines oder mehrerer anionischer Tensid(e), enthalten.
Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, sowie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.
Eine weitere Klasse bevorzugt eingesetzter nichtionischer Tenside, die entweder als alleiniges nichtionisches Tensid oder in Kombination mit anderen nicht-ionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester. Eine weitere Klasse von nichtionischen Tensiden, die vorteilhaft eingesetzt werden kann, sind die Alkylpolyglycoside (APG). Einsetzbare Alkypolyglycoside genügen der allgemeinen Formel RO(G)_{z}, in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen steht und G das Symbol ist, das für eine Glykoseeinheit mit 5 oder 6 C-Atomen, vorzugsweise für Glucose, steht. Der Glycosidierungsgrad z liegt dabei zwischen 1,0 und 4,0, vorzugsweise zwischen 1,0 und 2,0 und insbesondere zwischen 1,1 und 1,4. Bevorzugt eingesetzt werden lineare Alkylpolyglucoside, also Alkylpolyglycoside, in denen der Polyglycosylrest ein Glucoserest und der Alkylrest ein n-Alkylrest ist.

Die tensidhaltigen Zubereitungen können bevorzugt Alkylpolyglycoside enthalten, wobei Gehalte der für Wasch-, Spül- oder Reinigungszwecke vorgesehenen Zubereitungen an APG von über 0,2 Gew.-%, bezogen auf die gesamte Zubereitung, bevorzugt sind. Besonders bevorzugte tensidhaltige Zubereitungen enthalten APG in Mengen von 0,2 bis 10 Gew.-%, vorzugsweise in Mengen von 0,2 bis 5 Gew.-% und insbesondere in Mengen von 0,5 bis 3 Gew.-%.

Auch nichtionische Tenside vom Typ der Aminoxide, beispielsweise N-Kokos-alkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel (I), 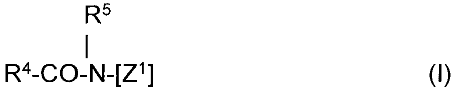 in der R⁴CO für einen aliphatischen Acylrest mit 6 bis 22 Kohlenstoffatomen, R⁵ für Wasserstoff, einen Alkyl- oder Hydroxyalkylrest mit 1 bis 4 Kohlenstoffatomen und [Z¹] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (II), 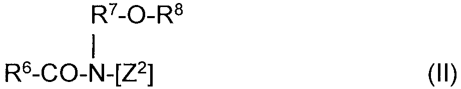 in der R⁶ für einen linearen oder verzweigten Alkyl- oder Alkenylrest mit 7 bis 12 Kohlenstoffatomen, R⁷ für einen linearen, verzweigten oder cyclischen Alkylrest oder einen Arylrest mit 2 bis 8 Kohlenstoffatomen und R⁸ für einen linearen, verzweigten oder cyclischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z²] für einen linearen Polyhydroxyalkylrest steht, dessen Alkylkette mit mindestens zwei Hydroxylgruppen substituiert ist, oder alkoxylierte, vorzugsweise ethoxylierte oder propxylierte Derivate dieses Restes.

[Z²] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können dann beispielweise, wie in der WO-A-95/07331 beschrieben, durch Umsetzung mit Fettsäuremethylestern in Gegenwart eines Alkoxids als Katalysator in die gewünschten Polyhydroxyfettsäureamide überführt werden.

Weiterhin kann es bevorzugt sein, neben anionischen und nichtionischen Tensiden auch kationische Tenside einzusetzen.

Als textilweichmachende Substanzen sind insbesondere kationische Tenside zu nennen. Beispiele kationische Tenside sind insbesondere quartäre Ammoniumverbindungen, kationische Polymere und Emulgatoren.

### Geeignete Beispiele sind quartäre Ammoniumverbindungen der Formeln (III) und (IV)

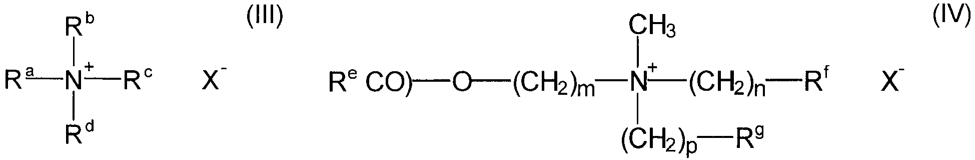

wobei in (IV) R^{a} und R^{b} für einen acyclischen Alkylrest mit 12 bis 24 Kohlenstoffatomen, R^{c} für einen gesättigten C₁-C₄ Alkyl- oder Hydroxyalkylrest steht, R^{d} entweder gleich R^{a}, R^{b} oder R^{c} ist oder für einen aromatischen Rest steht. X- steht entweder für ein Halogenid-, Metho-sulfat-, Methophosphat- oder Phosphation sowie Mischungen aus diesen. Beispiele für kationische Verbindungen der Formel (III) sind Didecyldimethylammoniumchlorid, Ditalgdimethylammoniumchlorid oder Dihexadecylammoniumchlorid.

Verbindungen der Formel (IV) sind sogenannte Esterquats. Esterquats zeichnen sich durch eine hervorragende biologische Abbaubarkeit aus. Hierbei steht R^{e} für einen aliphatischen Acylrest mit 12 bis 22 Kohlenstoffatomen mit 0, 1, 2 oder 3 Doppelbindungen; R^{f} steht für H, OH oder O(CO)R^{h}, R⁹ steht unabhängig von R^{f} für H, OH oder O(CO)Rⁱ, wobei R^{h} und Rⁱ unabhängig voneinander jeweils für einen aliphatischen Acylrest mit 12 bis 22 Kohlenstoffatomen mit 0, 1, 2 oder 3 Doppelbindungen steht. m, n und p können jeweils unabhängig voneinander den Wert 1, 2 oder 3 haben. X⁻ kann entweder ein Halogenid-, Methosulfat-, Methophosphat- oder Phosphation sowie Mischungen aus diesen sein. Bevorzugt sind Verbindungen, die für R^{f} die Gruppe O(CO)R^{h} und für R^{c} und R^{h} Alkylreste mit 16 bis 18 Kohlenstoffatomen enthalten. Besonders bevorzugt sind Verbindungen, bei denen R^{g} zudem für OH steht. Beispiele für Verbindungen der Formel (IV) sind Methyl-N-(2-hydroxyethyl)-N,N-di(talgacyl-oxyethyl)ammonium-methosulfat, Bis-(palmitoyl)-ethyl-hydroxyethyl-methyl-ammonium-methosulfat oder Methyl-N,N-bis(acyloxyethyl)-N-(2-hydroxyethyl)ammonium-methosulfat. Werden quarternierte Verbindungen der Formel (IV) eingesetzt, die ungesättigte Alkylketten aufweisen, sind die Acylgruppen bevorzugt, deren korrespondierenden Fettsäuren eine Jodzahl zwischen 5 und 80, vorzugsweise zwischen 10 und 60 und insbesondere zwischen 15 und 45 aufweisen und die ein cis/trans-Isomerenverhältnis (in Gew.-%) von größer als 30 : 70, vorzugsweise größer als 50 : 50 und insbesondere größer als 70 : 30 haben. Handelsübliche Beispiele sind die von Stepan unter der Marke Stepantex^{®} vertriebenen Methylhydroxyalkyldialkoyloxyalkylammoniummethosulfate oder die unter Dehyquart^{®} bekannten Produkte von Cognis bzw. die unter Rewoquat^{®} bekannten Produkte von Goldschmidt-Witco. Weitere bevorzugte Verbindungen sind die Diesterquats der Formel (V), die unter dem Namen Rewoquat^{®} W 222 LM bzw. CR 3099 erhältlich sind und neben der Weichheit auch für Stabilität und Farbschutz sorgen.

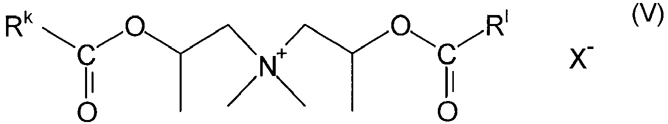

R^{k} und R^{l} stehen dabei unabhängig voneinander jeweils für einen aliphatischen Acylrest mit 12 bis 22 Kohlenstoffatomen mit 0, 1, 2 oder 3 Doppelbindungen.

Neben den oben beschriebenen quartären Verbindungen können auch andere bekannte Verbindungen eingesetzt werden, wie beispielsweise quartäre Imidazoliniumverbindungen der Formel (VI), 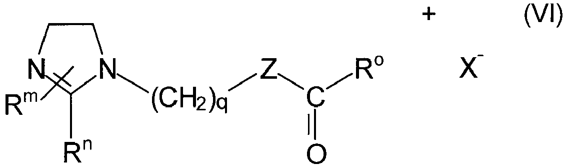 wobei R^{m} für H oder einen gesättigten Alkylrest mit 1 bis 4 Kohlenstoffatomen, Rⁿ und R^{o} unabhängig voneinander jeweils für einen aliphatischen, gesättigten oder ungesättigten Alkylrest mit 12 bis 18 Kohlenstoffatomen, Rⁿ alternativ auch für O(CO)R^{p} stehen kann, wobei R^{p} einen aliphatischen, gesättigten oder ungesättigten Alkylrest mit 12 bis 18 Kohlenstoffatomen bedeutet, und Z eine NH-Gruppe oder Sauerstoff bedeutet und X⁻ ein Anion ist. q kann ganzzahlige Werte zwischen 1 und 4 annehmen.

Weitere geeignete quartäre Verbindungen sind durch Formel (VII) beschrieben, 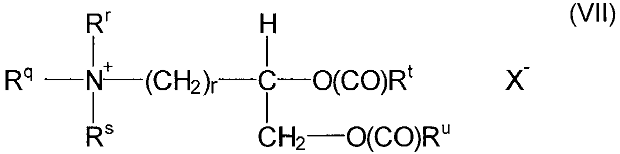 wobei R^{q}, R^{r} und R^{s} unabhängig voneinander für eine C₁₋₄-Alkyl-, Alkenyl- oder Hydroxyalkylgruppe steht, R^{t} und R^{u} jeweils unabhängig ausgewählt eine C₈₋₂₈-Alkylgruppe darstellt und r eine Zahl zwischen 0 und 5 ist.

Neben den Verbindungen der Formeln III bis VII können auch kurzkettige, wasserlösliche, quartäre Ammoniumverbindungen eingesetzt werden, wie Trihydroxyethylmethylammonium-methosulfat oder die Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid.

Auch protonierte Alkylaminverbindungen, die weichmachende Wirkung aufweisen, sowie die nicht quaternierten, protonierten Vorstufen der kationischen Emulgatoren sind geeignet.

Weitere erfindungsgemäß verwendbare kationische Verbindungen stellen die quaternisierten Proteinhydrolysate dar.

Zu den geeigneten kationischen Polymeren zählen die Polyquaternium-Polymere, wie sie im CTFA Cosmetic Ingredient Dictionary (The Cosmetic, Toiletry und Fragrance, Inc., 1997), insbesondere die auch als Merquats bezeichneten Polyquaternium-6-, Polyquaternium-7-, Polyquaternium-10-Polymere (Ucare Polymer IR 400; Amerchol), Polyquaternium-4-Copolymere, wie Pfropfcopolymere mit einen Cellulosegerüst und quartären Ammoniumgruppen, die über Allyldimethylammoniumchlorid gebunden sind, kationische Cellulosederivate, wie kationisches Guar, wie Guar-hydroxypropyltriammoniumchlorid, und ähnliche quaternierte Guar-Derivate (z. B. Cosmedia Guar, Hersteller: Cognis GmbH), kationische quartäre Zuckerderivate (kationische Alkylpolyglucoside), z. B. das Handelsprodukt Glucquat^{®}100, gemäß CTFA-Nomenklatur ein "Lauryl Methyl Gluceth-10 Hydroxypropyl Dimonium Chloride", Copolymere von PVP und Dimethylaminomethacrylat, Copolymere von Vinylimidazol und Vinylpyrrolidon, Aminosilicon-polymere und Copolymere,

Ebenfalls einsetzbar sind polyquaternierte Polymere (z. B. Luviquat Care von BASF) und auch kationische Biopolymere auf Chitinbasis und deren Derivate, beispielsweise das unter der Handelsbezeichnung Chitosan^{®} (Hersteller: Cognis) erhältliche Polymer.

Ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) Abil^{®}-Quat 3270 und 3272 (Hersteller: Goldschmidt-Rewo; diquartäre Polydimethylsiloxane, Quaternium-80), sowie Siliconquat Rewoquat^{®} SQ 1 (Tegopren^{®} 6922, Hersteller: Goldschmidt-Rewo).

### Ebenfalls einsetzbar sind Verbindungen der Formel (VIII),

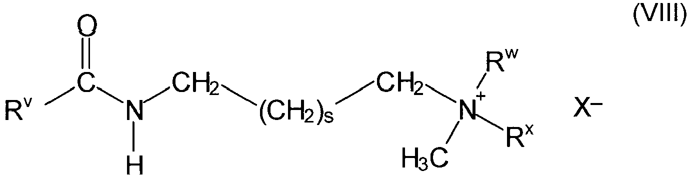

die Alkylamidoamine in ihrer nicht quaternierten oder, wie dargestellt, ihrer quaternierten Form, sein können. R^{v} kann ein aliphatischer Acylrest mit 12 bis 22 Kohlenstoffatomen mit 0, 1, 2 oder 3 Doppelbindungen sein. s kann Werte zwischen 0 und 5 annehmen. R^{w} und R^{x} stehen unabhängig voneinander jeweils für H, C₁₋₄-Alkyl oder Hydroxyalkyl. Bevorzugte Verbindungen sind Fettsäureamidoamine wie das unter der Bezeichnung Tego Amid^{®}S 18 erhältliche Stearylamidopropyldimethylamin oder das unter der Bezeichnung Stepantex^{®} X 9124 erhältliche 3-Talgamidopropyl-trimethylammonium-methosulfat, die sich neben einer guten konditionierenden Wirkung auch durch farbübertragungsinhibierende Wirkung sowie speziell durch ihre gute biologische Abbaubarkeit auszeichnen.
Werden kationische Tenside eingesetzt, so sind sie in den Zubereitungen bevorzugt in Mengen von 0,01 bis 10 Gew.-%, insbesondere von 0,1 bis 3,0 Gew.-% enthalten.

Der Gesamttensidgehalt kann in den für die erfindungsgemäße Verwendung beabsichtigten Mitteln zwischen 5 und 50 Gew.-%, bevorzugt zwischen 10 und 35 Gew.-% liegen. Neben den Tensiden sind Gerüststoffe die wichtigsten Inhaltsstoffe von Wasch- und Reinigungsmitteln. In den erfindunsgemäß verwendeten tensidhaltigen Zubereitungen können üblicherweise in Wasch- und Reinigungsmitteln eingesetzte Gerüststoffe enthalten sein, insbesondere also Zeolithe, Silicate, Carbonate, organische Cobuilder und - wo keine ökologischen Vorurteile gegen ihren Einsatz bestehen - auch die Phosphate.
Geeignete kristalline, schichtförmige Natriumsilicate besitzen die allgemeine Formel NaMSiₓO₂ₓ₊₁ ·H₂O, wobei M Natrium oder Wasserstoff bedeutet, x eine Zahl von 1,9 bis 4 und y eine Zahl von 0 bis 20 ist und bevorzugte Werte für x 2, 3 oder 4 sind. Derartige kristalline Schichtsilicate werden beispielsweise in der europäischen Patentanmeldung EP-A-0 164 514 beschrieben. Bevorzugte kristalline Schichtsilikate der angegebenen Formel sind solche, in denen M für Natrium steht und x die Werte 2 oder 3 annimmt. Insbesondere sind sowohl ß- als auch δ-Natriumdisilicate Na₂Si₂O₅ · yH₂O bevorzugt, wobei ß-Natriumdisilicat beispielsweise nach dem Verfahren erhalten werden kann, das in der internationalen Patentanmeldung WO-A-91/08171 beschrieben ist.
Einsetzbar sind auch amorphe Natriumsilicate mit einem Modul Na₂O : SiO₂ von 1 : 2 bis 1 : 3,3, vorzugsweise von 1 : 2 bis 1 : 2,8 und insbesondere von 1 : 2 bis 1 : 2,6, welche löseverzögert sind und Sekundärwascheigenschaften aufweisen. Die Löseverzögerung gegenüber herkömmlichen amorphen Natriumsilicaten kann dabei auf verschiedene Weise, beispielsweise durch Oberflächenbehandlung, Compoundierung, Kompaktierung/Verdichtung oder durch Übertrocknung hervorgerufen worden sein. Sogenannte röntgenamorphe Silicate, welche ebenfalls eine Löseverzögerung gegenüber den herkömmlichen Wassergläsern aufweisen, werden beispielsweise in der deutschen Patentanmeldung DE-A- 44 00 024 beschrieben. Die Produkte weisen mikrokristalline Bereiche der Größe 10 bis einige Hundert nm auf, wobei Werte bis max. 50 nm und insbesondere bis max. 20 nm bevorzugt sind. Insbesondere bevorzugt sind verdichtete/kompaktierte amorphe Silicate, compoundierte amorphe Silicate und übertrocknete röntgenamorphe Silicate.

Ein gegebenenfalls eingesetzter feinkristalliner, synthetischer und gebundenes Wasser enthaltender Zeolith ist vorzugsweise Zeolith A und/oder P. Als Zeolith des P-Typs wird Zeolith MAP (z. B. Handelsprodukt: Doucil A24 der Firma Crosfield) besonders bevorzugt. Geeignet sind jedoch auch Zeolith X sowie Mischungen aus A, X und/oder P. Kommerziell erhältlich und im Rahmen der vorliegenden Erfindung bevorzugt einsetzbar ist beispielsweise auch ein Co-Kristallisat aus Zeolith X und Zeolith A (ca. 80 Gew.-% Zeolith X), das von der Firma CONDEA Augusta S.p.A. unter dem Markennamen VEGOBOND AX^{®} vertrieben wird und durch die Formel

nNa₂O · (1-n)K₂O ·Al₂O₃ · (2 - 2,5)SiO₂ · (3,5 - 5,5) H₂O

beschrieben werden kann. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser.

Selbstverständlich ist in Waschmitteln auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate.

Brauchbare organische Gerüstsubstanzen sind beispielsweise die in Form ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern deren Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer Builderwirkung typischerweise auch die Eigenschaft einer Säuerungskomponente und dienen somit auch zur Einstellung eines niedrigeren und milderen pH-Wertes von tensidhaltigen Zubereitungen gemäß der Erfindung. Insbesondere sind in diesem Zusammenhang Citronensäure, Bernsteinsäure, Glutarsäure, Adipinsäure, Gluconsäure und beliebige Mischungen von diesen zu nennen.

Als Builder sind weiter polymere Polycarboxylate geeignet. Dies sind beispielsweise die Alkalimetallsalze der Polyacrylsäure oder der Polymethacrylsäure, beispielsweise solche mit einer relativen Molekülmasse von 500 bis 70.000 g/mol.

Bei den für polymere Polycarboxylate angegebenen Molmassen handelt es sich im Rahmen der vorliegenden Erfindung um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsäuren gemessenen Molmassen sind in der Regel deutlich höher als die im Rahmen der vorliegenden Erfindung angegebenen Molmassen.

Geeignete Polymere sind insbesondere Polyacrylate, die bevorzugt eine Molmasse von 2.000 bis 20.000 g/mol aufweisen. Aufgrund ihrer überlegenen Löslichkeit können aus dieser Gruppe wiederum die kurzkettigen Polyacrylate bevorzugt sein, die Molmassen von 2.000 bis 10.000 g/mol, besonders bevorzugt von 3.000 bis 5.000 g/mol, aufweisen.

Geeignet sind weiterhin copolymere Polycarboxylate, insbesondere solche der Acrylsäure mit Methacrylsäure oder der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molmasse, bezogen auf freie Säuren, beträgt im allgemeinen 2.000 bis 70.000 g/mol, vorzugsweise 20.000 bis 50.000 g/mol und insbesondere 30.000 bis 40.000 g/mol.

Die (co-)polymeren Polycarboxylate können entweder als Pulver oder als wäßrige Lösung eingesetzt werden. Der Gehalt an (co-)polymeren Polycarboxylaten in den erfindungsgemäßen Wasch- und Reinigungsmitteln beträgt vorzugsweise 0,5 bis 20 Gew.-%, insbesondere 3 bis 10 Gew.-%.

Zur Verbesserung der Wasserlöslichkeit können die Polymere auch Allylsulfonsäuren, Allyloxybenzolsulfonsäure und Methallylsulfonsäure als Monomer enthalten.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder Salze der Acrylsäure und der 2-Alkylallylsulfonsäure sowie Zucker-Derivate enthalten.

Weiter bevorzugte Copolymere sind solche, die als Monomere vorzugsweise Acrolein und Acrylsäure/Acrylsäuresalze bzw. Acrolein und Vinylacetat enthalten. Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate, die zum Teil neben Co-Builder-Eigenschaften auch eine bleichstabilisierende Wirkung aufweisen.

Weitere geeignete Buildersubstanzen sind Polyacetale, die durch Umsetzung von Dialdehyden mit Polyolcarbonsäuren erhalten werden können, die 5 bis 7 Kohlenstoffatome und mindestens 3 Hydroxygruppen aufweisen. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500.000 g/mol. Dabei ist ein Polysaccharid mit einem Dextrose-Äquivalent (DE) im Bereich von 0,5 bis 40, insbesondere von 2 bis 30, bevorzugt, wobei DE ein gebräuchliches Maß für die reduzierende Wirkung eines Polysaccharids im Vergleich zu Dextrose ist, welche ein DE von 100 besitzt. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2.000 bis 30.000 g/mol. Ein bevorzugtes Dextrin ist in der britischen Patentanmeldung 94 19 091 beschrieben.

Bei den oxidierten Derivaten derartiger Dextrine handelt es sich um deren Umsetzungsprodukte mit Oxidationsmitteln, die in der Lage sind, mindestens eine Alkoholfunktion des Saccharidrings zur Carbonsäurefunktion zu oxidieren. Ebenfalls geeignet ist ein oxidiertes Oligosaccharid, wobei ein an C₆ des Saccharidrings oxidiertes Produkt besonders vorteilhaft sein kann.

Auch Oxydisuccinate und andere Derivate von Disuccinaten, vorzugsweise Ethylendiamindisuccinat sind weitere geeignete Co-Builder. Dabei wird Ethylendiamin-N,N'-disuccinat (EDDS) bevorzugt in Form der Natrium- oder Magnesiumsalze verwendet. Weiterhin bevorzugt sind in diesem Zusammenhang auch Glycerindisuccinate und Glycerintrisuccinate. Geeignete Einsatzmengen liegen in zeolithhaltigen und/oder silicathaltigen Formulierungen bei 3 bis 15 Gew.-%.

Weitere brauchbare organische Co-Builder sind beispielsweise acetylierte Hydroxycarbonsäuren bzw. deren Salze, welche gegebenenfalls auch in Lactonform vorliegen können und welche mindestens 4 Kohlenstoffatome und wenigstens eine Hydroxygruppe sowie maximal zwei Säuregruppen enthalten.

Eine weitere Substanzklasse mit Co-Builder-Eigenschaften stellen die Phosphonate dar. Dabei handelt es sich insbesondere um Hydroxyalkan- bzw. Aminoalkanphosphonate. Unter den Hydroalkanphosphonaten ist das 1-Hydroxyethan-1,1-diphosphonat (HEDP) von besonderer Bedeutung als Co-Builder. Es wird vorzugsweise als Natriumsalz eingesetzt, wobei das Dinatriumsalz neutral und das Tetranatriumsalz alkalisch (pH = 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutralreagierenden Natriumsalze, z.B. als Hexanatriumsalz der EDTMP bzw. als Hepta- und Octanatriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die erfindungsgemäßen tensidhaltigen Zubereitungen auch Bleiche enthalten, bevorzugt sein, Aminoalkanphosphonate, insbesondere DTPMP, einzusetzen oder Mischungen aus den genannten Phosphonaten zu verwenden. Darüber hinaus können alle Verbindungen, die in der Lage sind, Komplexe mit Erdalkalimetallionen zu bilden, als Co-Builder eingesetzt werden.

Unter den als Bleichmittel dienenden, in Wasser H₂O₂ liefernden Verbindungen haben das Natriumperborat-tetrahydrat und das Natriumperborat-monohydrat besondere Bedeutung. Weitere brauchbare Bleichmittel sind beispielsweise Natriumpercarbonat, Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure. Werden Reinigungs- oder Bleichmittel-Zubereitungen für das maschinelle Geschirrspülen hergestellt, so können auch Bleichmittel aus der Gruppe der organischen Bleichmittel eingesetzt werden. Typische organische Bleichmittel sind die Diacylperoxide, wie z.B. Dibenzoylperoxid. Weitere typische organische Bleichmittel sind die Peroxysäuren, wobei als Beispiele besonders die Alkylperoxysäuren und die Arylperoxysäuren genannt werden. Bevorzugte Vertreter sind (a) die Peroxybenzoesäure und ihre ringsubstituierten Derivate, wie Alkylperoxybenzoesäuren, aber auch Peroxy-α-Naphtoesäure und Magnesiummonoperphthalat; (b) die aliphatischen oder substituiert aliphatischen Peroxysäuren, wie Peroxylaurinsäure, Peroxystearinsäure, ε-Phthalimidoperoxy-capronsäure [Phthaloiminoperoxyhexansäure (PAP)], o-Carboxybenzamido-peroxycapronsäure, N-Nonenylamidoperadipinsäure und N-Nonenylamidoper-succinate; und (c) aliphatische und araliphatische Peroxydicarbonsäuren, wie 1,12-Diperoxycarbonsäure, 1,9-Diperoxyazelainsäure, Diperocysebacinsäure, Diperoxybrassylsäure, die Diperoxyphthalsäuren, 2-Decyldiperoxybutan-1,4-disäure, N,N-Terephthaloyl-di(6-aminopercapronsäue) können eingesetzt werden.

Um beim Waschen oder Reinigen bei Temperaturen von 60 °C und darunter eine verbesserte Bleichwirkung zu erreichen, können Bleichaktivatoren in die tensidhaltigen Zubereitungen eingearbeitet werden. Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zusätzlich zu den konventionellen Bleichaktivatoren oder an deren Stelle können auch sogenannte Bleichkatalysatoren in die tensidhaltigen Zubereitungen eingearbeitet werden. Bei diesen Stoffen handelt es sich um bleichverstärkende Übergangsmetallsalze bzw. Übergangsmetallkomplexe wie beispielsweise Mn-, Fe-, Co-, Ru - oder Mo-Salenkomplexe oder -carbonylkomplexe. Auch Mn-, Fe-, Co-, Ru-, Mo-, Ti-, V- und Cu-Komplexe mit N-haltigen Tripod-Liganden sowie Co-, Fe-, Cu- und Ru-Amminkomplexe sind als Bleichkatalysatoren verwendbar.

Als Enzyme kommen solche aus der Klasse der Proteasen, Lipasen, Amylasen, Cellulasen bzw. deren Gemische in Frage. Besonders gut geeignet sind aus Bakterienstämmen oder Pilzen, wie Bacillus subtilis, Bacillus licheniformis und Streptomyces griseus gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase oder Protease und Cellulase oder aus Cellulase und Lipase oder aus Protease, Amylase und Lipase oder Protease, Lipase und Cellulase, insbesondere jedoch Cellulase-haltige Mischungen von besonderem Interesse. Auch Peroxidasen oder Oxidasen haben sich in einigen Fällen als geeignet erwiesen. Die Enzyme können an Trägerstoffen adsorbiert und/oder in Hüllsubstanzen eingebettet sein, um sie gegen vorzeitige Zersetzung zu schützen. Der Anteil der Enzyme, Enzymmischungen oder Enzymgranulate in den erfindungsgemäßen tensidhaltigen Zubereitungen kann beispielsweise etwa 0,1 bis 5 Gew.-%, vorzugsweise 0,1 bis etwa 2 Gew.-%, betragen.
Eine bevorzugte Gruppe geeigneter Additive sind optische Aufheller. Verwendet werden können hier die in Waschmitteln üblichen optischen Aufheller. Beispiele für optische Aufheller sind Derivate von Diaminostilbendisulfonsäure bzw. deren Alkalimetallsalze. Geeignet sind z. B. Salze der 4, 4'-Bis(2-anilino-4-morpholino1,3,5-triazinyl-6-amino-)stilben-2,2'-disulfonsäure oder gleichartig aufgebaute Verbindungen, die anstelle der Morpholino-Gruppe eine Diethanol-amino-Gruppe, eine Methylamino-Gruppe, eine Anilino-Gruppe oder eine 2-Methoxyethylamino-Gruppe tragen. Weiterhin können Aufheller vom Typ der substituierten Diphenylstyryle in den Teil-Portionen (waschaktiven Zubereitun-gen) der erfindungsgemäßen tensidhaltigen Zubereitungen enthalten sein, z. B. die Alkalisalze des 4,4'-Bis(2-sulfostyryl-)diphenyls, 4,4'-Bis(4-chlor-3-sulfostyryl-)diphenyls oder 4-(4-Chlorstyryl-)4'-(2-sulfostyryl-)diphenyls. Auch Gemische der vorgenannten Aufheller können verwendet werden.

Eine weitere bevorzugte Gruppe von Additiven sind UV-Schutz-Substanzen. UV-Absorber können auf die behandelten Textilien aufziehen und die Lichtbeständigkeit der Fasern und/oder die Lichtbeständigkeit des sonstiger Rezepturbestandteile verbessern. Unter UV-Absorber sind organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, wie beispielsweise das wasserlösliche Benzolsulfonsäure-3-(2H-benzotriazol-2-yl)-4-hydroxy-5-(methylpropyl)-mononatri umsalz (Cibafast^{®} H), in 3-Stellung Phenylsubstituierte Acrylate (Zimtsäurederivate), gegebenenfalls mit Cyanogruppen in 2-Stellung, Salicylate, organische Ni-Komplexe sowie Naturstoffe wie Umbelliferon und die körpereigene Urocansäure geeignet. Besondere Bedeutung haben Biphenyl- und vor allem Stilbenderivate wie sie beispielsweise in der EP 0728749 A beschrieben werden und kommerziell als Tinosorb^{®} FD oder Tinosorb^{®} FR ex Ciba erhältlich sind. Als UV-B-Absorber sind zu nennen 3-Benzylidencampher bzw. 3-Benzylidennorcampher und dessen Derivate, z.B. 3-(4-Methylbenzyliden)campher, wie in der EP 0693471 B1 beschrieben; 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzylester, Salicylsäurehomomenthylester; Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon; Ester der Benzalmalonsäure, vorzugsweise 4-Methoxybenzmalonsäuredi-2-ethylhexylester; Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB); Propan-1,3-dione, wie z.B. 1-(4-tert.Butylphenyl)-3-(4'methoxyphenyl)propan-1,3-dion; Ketotricyclo(5.2.1.0)decan-Derivate, wie in der EP 0694521 B1 beschrieben. Weiterhin geeignet sind 2-Phenylbenzimidazol-5-sulfonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hydroxy-4-methoxybenzophenon-5-sulfonsäure und ihre Salze; Sulfonsäurederivate des 3-Benzylidencamphers, wie z.B. 4-(2-Oxo-3-bornylidenmethyl)benzol-sulfonsäure und 2-Methyl-5-(2-oxo-3-bornyliden)sulfonsäure und deren Salze. Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beschrieben in der DE 19712033 A1 (BASF). Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. Vorzugsweise wird mikronisiertes Zinkoxid verwendet. Weitere geeignete UV-Lichtschutzfilter sind der Übersicht von P.Finkel in SÖFW-Journal 122, 543 (1996) zu entnehmen.
Die UV-Absorber werden üblicherweise in Mengen von 0,01 Gew.-% bis 5 Gew.-%, vorzugsweise von 0,03 Gew.-% bis 1 Gew.-%, eingesetzt.

Eine weitere bevorzugte Gruppe von Additiven sind Farbstoffe, insbesondere wasserlösliche oder wasserdispergierbare Farbstoffe. Bevorzugt sind hier Farbstoffe, wie sie zur Verbesserung der optischen Produktanmutung in den erfindungsgemäßen Wasch-, Spül-, Reinigungs- und Textilbehandlungsmitteln üblicherweise eingesetzt werden. Die Auswahl derartiger Farbstoffe bereitet dem Fachmann keine Schwierigkeiten, insbesondere da derartige übliche Farbstoffe eine hohe Lagerstabilität und Unempfindlichkeit gegenüber den übrigen Inhaltsstoffen der waschaktiven Zubereitungen und gegen Licht sowie keine ausgeprägte Substantivität gegenüber Textilfasern haben, um diese nicht anzufärben. Die Farbstoffe sind erfindungsgemäß in den Wasch- und/oder Reinigungsmitteln gemäß der Erfindung in Mengen von unter 0,01 Gew.-% zugegen.

Eine weitere Klasse von Additiven, die den Wasch- und/oder Reinigungsmitteln zugesetzt werden kann, sind Polymere. Unter diesen Polymeren kommen zum einen Polymere in Frage, die beim Waschen oder Reinigen bzw. Spülen Cobuilder-Eigenschaften zeigen, also zum Beispiel Polyacrylsäuren, auch modifizierte Polyacrylsäuren oder entsprechende Copolymere. Eine weitere Gruppe von Polymeren sind Polyvinylpyrrolidon und andere Vergrauungsinhibitoren, wie Copolymere von Polyvinylpyrrolidon, Cellulose-Ether und dergleichen. Weiterhin kommen als Polymere bevorzugt auch sogenannte Soil Repellents in Frage, wie sie nachfolgend im einzelnen beschrieben werden.

Als weitere Zusätze können die Wasch- und Reinigungsmittel auch sog. Soil Repellents enthalten, also Polymere, die auf Fasern aufziehen, die Öl- und Fettauswaschbarkeit aus Textilien positiv beeinflussen und damit einer Wiederanschmutzung gezielt entgegenwirken. Dieser Effekt wird besonders deutlich, wenn ein Textil verschmutzt wird, das bereits vorher mehrfach mit einem erfindungsgemäßen Wasch- oder Reinigungsmittel, das diese öl- und fettlösende Komponente enthält, gewaschen wurde. Zu den bevorzugten öl- und fettlösenden Komponenten zählen beispielsweise nichtionische Celluloseether wie Methylcellulose und Methylhydroxypropylcellulose mit einem Anteil an Methoxy-Gruppen von 15 bis 30 Gew.-% und an Hydroxypropoxy-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten, insbesondere Polymere aus Ethylenterephthalaten und/oder Polyethylenglykolterephthalaten oder anionisch und/oder nichtionisch modifizierten Derivaten von diesen. Besonders bevorzugt von diesen sind die sulfonierten Derivate der Phthalsäure- und der Terephthalsäure-Polymere. Insbesondere wenn es sich um flüssige oder gelförmige Zubereitungen handelt, können diese auch Lösungsmittel enthalten. Beispiele für geeignete Lösungsmittel sind ein- oder mehrwertige Alkohole mit 1 bis 4 C-Atomen. Bevorzugte Alkohole sind Ethanol, 1,2-Propandiol, Glycerin sowie deren beliebigen Gemische. Die Lösungsmittel können in flüssigen Zubereitungen in einer Menge von 2 bis 12 Gew.-%, bezogen auf die fertige Zubereitung, enthalten sein.
Die genannten Additive werden den Wasch- und/oder Reinigungsmitteln in Mengen bis höchstens 30 Gew.-%, vorzugsweise 2 bis 20 Gew.-%, zugesetzt.
Diese Aufzählung von Wasch- und Reinigungsmittelinhaltsstoffen, die in den erfindungsgemäß verwendeten" Wasch-, Spül- oder Reinigungsmittel vorkommen können, ist keineswegs abschließend, sondern gibt lediglich die wesentlichen typischen Inhaltsstoffe derartiger Mittel wieder. Insbesondere können, soweit es sich um flüssige oder gelförmige Zubereitungen handelt, in den Mitteln auch organische Lösungsmittel enthalten sein. Vorzugsweise handelt es sich um ein- oder mehrwertige Alkohole mit 1 bis 4 C-Atomen. Bevorzugte Alkohole in solchen Mitteln sind Ethanol, 1,2-Propandiol, Glycerin sowie Gemische aus diesen Alkoholen. In bevorzugten Ausführungsformen enthalten derartige Mittel 2 bis 12 Gew.-% solcher Alkohole.

Nach einer besonderen Ausführungsform ist insbesondere die Verwendung flüssiger oder fester Waschmittel bevorzugt. Ebenfalls besonders bevorzugt ist die Verwendung von Wasch- und Reinigungsmitteln die für die Feinwäsche bzw. schonende Behandlung von empfindlichen Textilien geeignet sind.
Insbesondere sind auch Textilpflegemittel, insbesondere Textilnachbehandlungsmittel, bevorzugt Textilkonditioniermittel, Weichspüler oder Trocknertücher geeignet, die Patchouliöl, Patchoulialkohol und/oder dessen Derivate enthalten.
Je nach gewünschtem Verwendungszweck können weitere Inhaltsstoffe eingesetzt werden. Weichspülerzusammensetzungen für die Spülbadavivage sind im Stand der Technik breit beschrieben. Üblicherweise enthalten diese Zusammensetzungen als Aktivsubstanz eine kationische quartäre Ammoniumverbindung, die in Wasser dispergiert wird. Je nach Gehalt der fertigen Weichmacherzusammensetzung an Aktivsubstanz spricht man von verdünnten, anwendungsfertigen Produkten (Aktivsubstanzgehalte unter 7 Gew.-%) oder sogenannten Konzentraten (Aktivsubstanzgehalt über 7 Gew.-%). Wegen des geringeren Volumens und den damit gleichzeitig verringerten Verpackungs- und Transportkosten besitzen die Textilweichmacherkonzentrate Vorteile aus ökologischer Sicht und haben sich im Markt mehr und mehr durchgesetzt. Aufgrund der Einarbeitung von kationischen Verbindungen, die nur eine geringe Wasserlöslichkeit aufweisen, liegen übliche Weichspülerzusammensetzungen in Form von Dispersionen vor, besitzen ein milchig-trübes Aussehen und sind nicht durchscheinend. Aus Gründen der Produktästhetik kann es aber auch gewünscht sein, dem Verbraucher durchscheinende, klare Weichspüler zur Verfügung zu stellen, die sich optisch von den bekannten Produkten abheben.

Als textilweichmachende Aktivsubstanz enthalten erfindungsgemäß verwendete Weichspüler vorzugsweise kationische Tenside, die bereits weiter oben ausführlich beschrieben wurden. Besonders bevorzugt enthalten diese erfindungsgemäße Mittel sogenannte Esterquats. Während es eine Vielzahl möglicher Verbindungen aus dieser Substanzklasse gibt, werden erfindungsgemäß mit besonderem Vorzug Esterquats eingesetzt, die sich durch Umsetzung von Trialkanolaminen mit einer Mischung aus Fettsäuren und Dicarbonsäuren, gegebenenfalls nachfolgende Alkoxylierung des Reaktionsproduktes und Quaternierung in an sich bekannter Weise herstellen lassen, wie es in der DE 195 39 846 beschrieben ist.
Die auf diese Weise hergestellten Esterquats eignen sich in hervorragender Weise zur Herstellung Portionen, die als Weichspüler eingesetzt werden können. Da je nach Wahl des Trialkanolamins, der Fettsäuren und der Dicarbonsäuren sowie des Quaternierungsmittels eine Vielzahl geeigneter Produkte hergestellt und in den erfindungsgemäß eingesetzten Mitteln eingesetzt werden kann, ist eine Beschreibung der erfindungsgemäß vorzugsweise einzusetzenden Esterquats über ihren Herstellungsweg präziser als die Angabe einer allgemeinen Formel.
Die genannten Komponenten, die miteinander zu den vorzugsweise einzusetzenden Esterquats reagieren, können in variierenden Mengenverhältnissen zueinander eingesetzt werden. Im Rahmen der vorliegenden Erfindung sind Weichspüler bevorzugt, in denen ein Umsetzungsprodukt von Trialkanolaminen mit einer Mischung aus Fettsäuren und Dicarbonsäuren im molaren Verhältnis 1:10 bis 10:1, vorzugsweise 1:5 bis 5:1, das gegebenenfalls alkoxyliert und anschließend in an sich bekannter Weise quaterniert wurde, in Mengen von 2 bis 60, vorzugsweise 3 bis 35 und insbesondere 5 bis 30 Gew.-% enthalten ist. Besonders bevorzugt ist dabei die Verwendung von Triethanolamin, so daß weitere bevorzugte Weichspüler der vorliegenden Erfindung ein Umsetzungsprodukt von Triethanolamin mit einer Mischung aus Fettsäuren und Dicarbonsäuren im molaren Verhältnis 1:10 bis 10:1, vorzugsweise 1:5 bis 5:1, das gegebenenfalls alkoxyliert und anschließend in an sich bekannter Weise quaterniert wurde, in Mengen von 2 bis 60, vorzugsweise 3 bis 35 und insbesondere 5 bis 30 Gew.-% enthalten.
Als Fettsäuren können im Reaktionsgemisch zur Herstellung der Esterquats sämtliche aus pflanzlichen oder tierischen Ölen und Fetten gewonnenen Säuren verwendet werden. Dabei kann im Rekationsgemisch als Fettsäure durchaus auch eine bei Raumtemperatur nicht-feste, d.h. pastöse bis flüssige, Fettsäure eingesetzt werden.
Die Fettsäuren können unabhängig von ihrem Aggregatzustand gesättigt oder einbis mehrfach ungesättigt sein. Selbstverständlich können nicht nur "reine" Fettsäuren eingesetzt werden, sondern auch die bei der Spaltung aus Fetten und Ölen gewonnenen technischen Fettsäuregemische, wobei diese Gemische aus ökonomischer Sicht wiederum deutlich bevorzugt sind.
So lassen sich in den Reaktionsmischungen zur Herstellung der Esterquats für die erfindungsgemäß verwendeten klaren wäßrigen Weichspüler beispielsweise einzelne Spezies oder Gemische folgender Säuren einsetzen: Caprylsäure, Pelargonsäure, Caprinsäure, Laurinsäure, Myristinsäure, Palmitinsäure, Stearinsäure, Octadecan-12-ol-säure, Arachinsäure, Behensäure, Lignocerinsäure, Cerotinsäure, Melissinsäure, 10-Undecensäure, Petroselinsäure, Petroselaidinsäure, Ölsäure, Elaidinsäure, Ricinolsäure, Linolaidinsäure, α- und β-Eläosterainsäure, Gadoleinsäure, Erucasäure, Brassidinsäure. Selbstverständlich sind auch die Fettsäuren mit ungerader Anzahl von C-Atomen einsetzbar, beispielsweise Undecansäure, Tridecansäure, Pentadecansäure, Heptadecansäure, Nonadecansäure, Heneicosansäure, Tricosansäure, Pentacosansäure, Heptacosansäure.
Im Rahmen der vorliegenden Erfindung ist die Verwendung von Fettsäuren der Formel XIII im Reaktionsgemisch zur Herstellung der Esterquats bevorzugt, so daß bevorzugte Weichspüler ein Umsetzungsprodukt von Trialkanolaminen mit einer Mischung aus Fettsäuren der Formel IX,

R¹-CO-OH (IX)

in der R1-CO- für einen aliphatischen, linearen oder verzweigten Acylrest mit 6 bis 22 Kohlenstoffatomen und 0 und/oder 1, 2 oder 3 Doppelbindungen steht und Dicarbonsäuren im molaren Verhältnis 1:10 bis 10:1, vorzugsweise 1:5 bis 5:1, das gegebenenfalls alkoxyliert und anschließend in an sich bekannter Weise quaterniert wurde, in Mengen von 2 bis 60, vorzugsweise 3 bis 35 und insbesondere 5 bis 30 Gew.-% in den Mitteln enthalten.

Als Dicarbonsäuren, die sich zur Herstellung der in den erfindungsgemäß verwendeten Mitteln einzusetzenden Esterquats eignen, kommen vor allem gesättigte oder ein- bzw. mehrfach ungesättigte α,ωDicarbonsäuren in Betracht. Beispielhaft seien hier die gesättigten Spezies Oxalsäure, Malonsäure, Bernsteinsäure, Glutarsäure, Adipinsäure, Pimelinsäure, Korksäure, Azelainsäure, Sebacinsäure, Undecan- und Dodecansäure, Brassylsäure, Tetra- und Pentadecansäure, Thapisäure sowie Hepta-, Octa- und Nonadecansäure, Eicosan- und Heneicosansäure sowie Phellogensäure genannt. Vorzugsweise im Reaktionsgemisch eingesetzt werden dabei Dicarbonsäuren, die der allgemeinen Formel XIII folgen, so daß erfindungsgemäße Mittel bevorzugt sind, die ein Umsetzungsprodukt von Trialkanolaminen mit einer Mischung aus Fettsäuren und Dicarbonsäuren der Formel X,

HO-OC-[X]-CO-OH (X)

in der X für eine gegebenenfalls hydroxysubstituierte Alkylengruppe mit 1 bis 10 Kohlenstoffatomen steht, im molaren Verhältnis 1:10 bis 10:1, vorzugsweise 1:5 bis 5:1, das gegebenenfalls alkoxyliert und anschließend in an sich bekannter Weise quaterniert wurde, in Mengen von 2 bis 60, vorzugsweise 3 bis 35 und insbesondere 5 bis 30 Gew.-% in den Mitteln enthalten.

Unter der Vielzahl der herstellbaren und erfindungsgemäß einsetzbaren Esterquats haben sich wiederum solche besonders bewährt, in denen das Alkanolamin Treithanolamin und die Dicarbonsäure Adipinsäure ist. Somit sind im Rahmen der vorliegenden Erfindung Mittel besonders bevorzugt, die ein Umsetzungsprodukt von Triethanolamin mit einer Mischung aus Fettsäuren und Adipinsäure im molaren Verhältnis 1:5 bis 5:1, vorzugsweise 1:3 bis 3:1, das anschließend in an sich bekannter Weise quaterniert wurde, in Mengen von 2 bis 60, vorzugsweise 3 bis 35 und insbesondere 5 bis 30 Gew.-% in den Mitteln enthalten

Die bei der erfindungsgemäßen Verwendung eingesetzten Mittel können - und abhängig davon, ob sie als Textilwaschmittel, Waschhilfsmittel oder Weichspüler formuliert werden - auch mit weiteren Zusatznutzen ausgestattet werden. Hier sind beispielsweise farbübertragungsinhibierende Zusammensetzungen, Mittel mit "Anti-Grau-Formel", Mittel mit Bügelerleichterung, Mittel mit besonderer Duftfreisetzung, Mittel mit verbesserter Schmutzablösung bzw. Verhinderung von Wiederanschmutzung, antibakterielle Mittel, UV-Schutzmittel, farbauffrischende Mittel usw. formulierbar. Einige Beispiele werden nachstehend erläutert:
Da textile Flächengebilde, insbesondere aus Reyon, Zellwolle, Baumwolle und deren Mischungen, zum Knittern eigen können, weil die Einzelfasern gegen Durchbiegen, Knicken. Pressen und Quetschen quer zur Faserrichtung empfindlich sind, können die bei der erfindungsgemäßen Verwendung eingesetzten Mittel synthetische Knitterschutzmittel enthalten. Hierzu zählen beispielsweise synthetische Produkte auf der Basis von Fettsäuren, Fettsäureestern. Fettsäureamiden, -alkylolestern, -alkylolamiden oder Fettalkoholen, die meist mit Ethylenoxid umgesetzt sind, oder Produkte auf der Basis von Lecithin oder modifizierter Phosphorsäureester.

Ein erhöhter Tragekomfort kann aus der zusätzlichen Verwendung von Antistatika resultieren, die den bei der erfindungsgemäßen Verwendung eingesetzten Mitteln zusätzlich beigefügt werden. Antistatika vergrößern die Oberflächenleitfähigkeit und ermöglichen damit ein verbessertes Abfließen gebildeter Ladungen. Äußere Antistatika sind in der Regel Substanzen mit wenigstens einem hydrophilen Molekülliganden und geben auf den Oberflächen einen mehr oder minder hygroskopischen Film. Diese zumeist grenzflächenaktiven Antistatika lassen sich in stickstoffhaltige (Amine, Amide, quartäre Ammoniumverbindungen), phosphorhaltige (Phosphorsäureester) und schwefelhaltige (Alkylsulfonate, Alkylsulfate) Antistatika unterteilen. Lauryl- (bzw. Stearyl-) dimethylbenzylammoniumchloride eignen sich als Antistatika für Textilien bzw. als Zusatz zu Waschmitteln, wobei zusätzlich ein Avivageeffekt erzielt wird.

Zur Verbesserung des Wasserabsorptionsvermögens, der Wiederbenetzbarkeit der behandelten Textilien und zur Erleichterung des Bügelns der behandelten Textilien können in den bei der erfindungsgemäßen Verwendung eingesetzten Mitteln beispielsweise Silikonderivate eingesetzt werden. Diese verbessern zusätzlich das Ausspülverhalten der bei der erfindungsgemäßen Verwendung eingesetzten Mittel durch ihre schauminhibierenden Eigenschaften. Bevorzugte Silikonderivate sind beispielsweise Polydialkyl- oder Alkylarylsiloxane, bei denen die Alkylgruppen ein bis fünf C-Atome aufweisen und ganz oder teilweise fluoriert sind. Bevorzugte Silikone sind Polydimethylsiloxane, die gegebenenfalls derivatisiert sein können und dann aminofunktionell oder quaterniert sind bzw. Si-OH-, Si-H- und/oder Si-Cl-Bindungen aufweisen. Die Viskositäten der bevorzugten Silikone liegen bei 25°C im Bereich zwischen 100 und 100.000 Centistokes, wobei die Silikone in Mengen zwischen 0,2 und 5 Gew.-%, bezogen auf das gesamte Mittel eingesetzt werden können.

Schließlich können die Mittel auch UV-Absorber enthalten, die auf die behandelten Textilien aufziehen und die Lichtbeständigkeit der Fasern verbessern. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate), gegebenenfalls mit Cyanogruppen in 2-Stellung, Salicylate, organische Ni-Komplexe sowie Naturstoffe wie Umbelliferon und die körpereigene Urocansäure geeignet.
Ein weitere Gegenstande der vorliegenden Erfindung ist die Verwendung von Patchouliöl, Patchoulialkohol und/oder Ester und Ether von Patchoulialkohol, Patchoulenol, Norpatchoulenol und Seychellen zur Ausrüstung von Verpackungen, insbesondere solchen für Lebensmittel, Filtermedien, Baustoffe, Bauhilfsstoffe, Textilien, Pelze, Papier, Felle oder Leder zur Hemmung der asexuellen Vermehrung von humanpathogenen Pilzen oder zur Verminderung der Anhaftung von Bakterien oder humanpathogenen Pilzen.

Die Ausrüstung des Papiers bzw. der Verpackungen, Oberflächen, Textilien, Pelze, Felle oder Leder erfolgt in dem Fachmann bekannter Weise, beispielsweise durch Eintauchen oder Besprühen des Papiers oder der Textilien, Pelze, Felle oder Leder in eine geeignet konzentrierte Lösung eines erfindungsgemäßen Mittels. So können beispielsweise auch Kunstwerke auf Papier, Pergament, Holz und/oder Leinwand vor dem Befall durch Mikroorganismen, insbesondere Schimmelbefall, geschützt bzw. von diesem befreit werden. Die Ausrüstung der Filtermedien, Baustoffe oder Bauhilfsstoffe erfolgt beispielsweise durch mechanisches Einarbeiten oder Aufbringen einer geeignet konzentrierten Lösung eines Mittels in bzw. auf die Filtermedien, Baustoffe oder Bauhilfsstoffe.

Patchouliöl und Lösungen von Patchoulialkohol, vorzugsweise in organischen Lösungsmitteln, können vorteilhafterweise besonders gut auf bzw. in solche Bau- und Bauhilfsstoffe aufgebracht bzw. eingearbeitet werden. Eine nachträgliche Ausrüstung der Bau- bzw. Bauhilfsstoffe oder eine Wiederaufladung nach längerem Gebrauch bereits ausgerüsteter Bau- bzw. Bauhilfsstoffe, beispielsweise bei Dichtungsmassen, durch Auftragen der erfindungsgemäßen Mittel ist daher problemlos möglich.

Vorzugsweise sind die erfindungsgemäß ausgerüsteten Baustoffe und/oder Bauhilfsstoffe ausgewählt unter Klebe-, Dichtungs-, Spachtel- und Anstrichmassen, Kunststoffen, Lacken, Farben, Putz, Mörtel, Estrich, Beton, Isoliermaterialien sowie Grundierungen. Besonders bevorzugte Baustoffe oder Bauhilfsstoffe sind Fugendichtungsmassen (bspw. silikonhaltige Fugendichtungsmassen), Tapetenkleister, Putz, Teppichfixierer, Silikonkleber, Fliesenkleber, Dispersionsfarben und Anstrichmassen für den Außen- und/oder Innenbereich.

Dichtungsmassen und insbesondere Fugendichtungsmassen enthalten typischerweise organische Polymere sowie in vielen Fällen mineralische oder organische Füllstoffe und sonstige Additive.

Geeignete Polymere sind beispielsweise thermoplastische Elastomere, wie in der DE-A-3602526 der Anmelderin beschrieben, vorzugsweise Polyurethane und Acrylate. Geeignete Polymere sind auch in den Offenlegungsschriften DE-A-3726547, DE-A-4029504 und DE-A-4009095 der Anmelderin sowie in DE-A-19704553 und DE-A-4233077 genannt, auf die hiermit in vollem Umfang Bezug genommen wird.

Dichtungsmassen und insbesondere Fugendichtungsmassen enthalten typischerweise organische Polymere sowie in vielen Fällen mineralische oder organische Füllstoffe und sonstige Additive.

Geeignete Polymere sind beispielsweise thermoplastische Elastomere, wie in der DE-A-3602526 der Anmelderin beschrieben, vorzugsweise Polyurethane und Acrylate. Geeignete Polymere sind auch in den Offenlegungsschriften DE-A-3726547, DE-A-4029504 und DE-A-4009095 der Anmelderin sowie in DE-A-19704553 und DE-A-4233077 genannt, auf die hiermit in vollem Umfang Bezug genommen wird.

Die erfindungsgemäßen Dichtungsmassen (Dichtstoffe bzw. Dichtstoffmischungen) enthalten bevorzugt 0,0001 - 1,5 Gew.-% Patchouliöl, Patchoulialkohol und/oder dessen Derivate. Besonders bevorzugt sind Bereiche zwischen 0,001 und 1,0 Gew.-%, insbesondere bis 0,5 Gew.-%.

Erfindungsgemäß kann die Ausrüstung der erfindungsgemäßen Dichtstoffe sowohl im ungehärteten oder bei unter 60 °C gehärteten Zustand erfolgen. Dichtstoffe sind im erfindungsgemäßen Zusammenhang Materialien gemäß DIN EN 26927, insbesondere solche, die plastisch oder elastisch als Dichtstoffe aushärten. Die erfindungsgemäßen Dichtstoffe können alle für die entsprechenden Dichtungsmassen typischen Zusatzstoffe, wie z.B. typische Verdickungsmittel, verstärkende Füllstoffe, Vernetzer, Vernetzungskatalysatoren, Pigmente, Haftmittel oder sonstige Volumenextender enthalten. Dichtstoffe enthaltend Patchouliöl, Patchoulialkohol und/oder dessen Derivate können durch Eindispergieren in dem Fachmann bekannter Weise z.B. durch die Verwendung von Dispergiereinrichtungen, Kneter, Planetenmischer .usw., unter Ausschluss von Feuchtigkeit und Sauerstoff sowohl in die fertige als auch in Teile dieser Dichtungsmassen bzw. zusammen mit einer oder mehreren Komponenten der Dichtungsmassen eingearbeitet werden.

Selbst die Behandlung von bereits ausgehärteten, vernetzten Dichtungsmassenoberflächen kann durch Aufbringen von Lösungen bzw. Suspensionen der erfindungsgemäß verwendeten Substanz durchgeführt werden, indem der Wirkstoff durch Quellung bzw. Diffusion in die Dichtungsmasse transportiert wird.

Erfindungsgemäß einsetzbare Dichtstoffe können sowohl auf Silikon-, Urethan- als auch Acrylbasis hergestellt werden. Dichtstoffe auf Urethanbasis sind bspw. in Ullmann's Encyclopedia of Industrial Chemistry (8. Auflage 2003, Kapitel 4) sowie US 4,417,042 offenbart.

Silikondichtstoffe sind dem Fachmann bekannt, bspw. aus der EP 0 118 030 A, EP 0 316 591 A, EP 0 327 847 A, EP 0 553 143 A, DE 195 49 425 A und US 4,417,042.

Beispiele für Acryldichtstoffe sind u.a. in der WO 01/09249 oder der US 5,077,360 offenbart.

Insbesondere sind bei Raumtemperatur vernetzende Systeme, wie bspw. in der EP 0 327 847 oder US 5,077,360 beschrieben, bevorzugt. Dabei kann es sich um ein- oder mehrkomponentige Systeme handeln, wobei in den mehrkomponentigen Systemen Katalysator und Vernetzer getrennt vorliegen können (beispielsweise offenbart in den Patentschriften US 4,891,400 und US 5,502,144), oder andere sogenannte Silikon RVT 2K-Systeme, insbesondere platinfreie Systeme.

Besonders bevorzugt sind sogenannte Einkomponentensysteme, die alle Inhaltsstoffe zum Aufbau einer Dichtungsmasse enthalten, unter Abschluß von Luftfeuchtigkeit und/oder Luftsauerstoff gelagert werden und am Einsatzort unter Reaktion mit dem Luftsauerstoff aushärten. Besonders bevorzugt sind die sogenannten Silikon-Neutralsysteme, in denen die Umsetzung von Vernetzern mit dem Wasser der Umgebungsluft nicht zu korrosiven, sauren, basischen oder geruchsintensiven Spaltprodukten führt. Beispiele für solche Systeme sind in der DE 195 49 425, der US 4,417,042 oder der EP 0 327 847 offenbart.

Die Dichtungsmassen und insbesondere Fugendichtungsmassen können wäßrige oder organische Lösungsmittel enthalten. Als organische Lösungsmittel kommen Kohlenwasserstoffe wie Cyclohexan, Toluol oder auch Xylol oder Petrolether in Frage. Weitere Lösungsmittel sind Ketone wie Methylbutylketon oder Chlorkohlenwasserstoffe.

Weiterhin können die Dichtungsmassen noch weitere kautschukartige Polymere enthalten. Hier kommen relativ niedermolekulare, handelsübliche Typen von Polyisobutylen, Polyisopren oder auch Polybutadienstyrol in Frage. Auch die Mitverwendung von abgebautem Naturkauschuk oder von Neoprenkautschuk ist möglich. Hier können auch bei Raumtemperatur noch fließfähige Typen eingesetzt werden, welche häufig als "Flüssigkautschuk" bezeichnet werden.

Die erfindungsgemäßen Dichtungsmassen können verwendet werden, um die verschiedensten Materialien miteinander zu verbinden bzw. abzudichten. Hier ist in erster Linie an die Verwendung auf Beton, auf Glas, auf Putz und/oder Emaille sowie Keramik und Porzellan gedacht. Aber auch das Verbinden bzw. Abdichten von Formteilen bzw. Profilen aus Aluminium, Stahl, Zink oder auch aus Kunststoffen wie PVC oder Polyurethanen oder Acrylharzen ist möglich. Schließlich sei das Abdichten von Holz oder Holzmaterialien mit den verschiedensten anderen Werkstoffen erwähnt.

Die Standfestigkeit von Fugendichtungsmassen wird in der Regel durch Zusatz von feinteiligen Feststoffen - auch Füllstoffe genannt - erzielt. Diese lassen sich in solche organischer und solche anorganischer Art unterscheiden. Als anorganische Füllstoffe können beispielsweise Kieselsäure/Siliciumdioxid (gecoatet oder ungecoatet), Kreide - gecoatet oder ungecoatet - und/oder Zeolithe bevorzugt sein. Letztere können zudem auch als Trockenmittel fungieren. Als organischer Füllstoff kommt z. B. PVC- Pulver in Betracht. Die Füllstoffe tragen im allgemeinen wesentlich dazu bei, dass die Dichtungsmasse nach der Anwendung einen notwendigen inneren Halt besitzt, so dass ein Auslaufen oder Ausbuchten der Dichtungsmasse aus senkrechten Fugen verhindert wird. Die genannten Zusatz- bzw. Füllstoffe lassen sich in Pigmente und thixotropierende Füllstoffe, auch verkürzt als Thixotropiermittel bezeichnet, einteilen.

Als Thixotropierungsmittel eignen sich die bekannten Thixotropierungsmittel wir Bentone, Kaoline oder auch organische Verbindungen wie hydriertes Rizinusöl bzw. Derivate desselben mit mehrfunktionellen Aminen oder die Umsetzungsprodukte von Stearinsäure oder Rizinolsäure mit Ethylendiamin. Als besonders günstig hat sich die Mitverwendung von Kieselsäure, insbesondere von Kieselsäure aus der Pyrolyse erwiesen. Außerdem kommen als Thixotropiermittel im wesentlichen quellfähige Polymerpulver in Betracht. Beispiele sind hierfür Polyacrylnitril, Polyurethan, Polyvinylchlorid, Polyacrylsäureester, Polyvinylalkohole, Polyvinylacetate sowie die entsprechenden Copolymerisate. Besonders gute Ergebnisse lassen sich mit feinteiligem Polyvinylchloridpulver erhalten. Neben den Thixotropierungsmitteln können auch noch zusätzlich Haftvermittler eingesetzt werden wie etwa Mercaptoalkylsilan. Hier hat es sich als zweckmäßig erwiesen, ein Monomercaptoalkyltrialkoxysilan einzusetzen. Handelsüblich ist beispielsweise das Mercaptopropyltrimethoxysilan.

Die Eigenschaften einer Fugendichtungsmasse lassen sich noch weiter verbessern, wenn dem als Thixotropiermittel verwendeten Kunststoffpulver weitere Komponenten zugesetzt werden. Dabei handelt es sich um Stoffe, die unter die Kategorie der für Kunststoffe angewendeten Weichmacher bzw. Quellmittel und Quellhilfsmittel fallen.

Es kommen z. B. Weichmacher, insbesondere für die Dichtungsmassen auf Urethan- bzw. Acrylbasis aus der Klasse der Phthalsäureester in Betracht. Beispiele für anwendbare Verbindungen aus dieser Substanzklasse sind Dioctylphthalat, Dibutylphthalat und Benzylbutylphthalat. Weitere geeignete Substanzklassen sind Chlorparaffine, Alkylsulfonsäureester etwa der Phenole oder Kresole sowie Fettsäureester.

Für die Silikondichtungsmassen sind als Weichmacher Silikonöle, besonders bevorzugt Polydimethylsiloxane, sowie Kohlenwasserstoffe und/oder deren Gemische, von denen besonders Kohlenwasserstoffe bzw. deren Gemische mit einem Siedepunkt von größer 200°C, insbesondere größer 230 °C, gut geeignet.

Als Quellhilfsmittel sind solche niedermolekularen organischen Substanzen einsetzbar, die mit dem Polymerpulver und dem Weichmacher mischbar sind. Derartige Quellhilfsmittel lassen sich den einschlägigen Kunststoff- und Polymer-Handbüchern für den Fachmann entnehmen. Als bevorzugte Quellhilfsmittel für Polyvinylchloridpulver dienen Ester, Ketone, aliphatische Kohlenwasserstoffe, aromatische Kohlenwasserstoffe sowie aromatische Kohlenwasserstoffe mit Alkylsubstituenten.

Als Pigmente und Farbstoffe werden die für diese Verwendungszwecke bekannten Substanzen wie Titandioxid, Eisenoxide und Ruß verwendet

Zur Verbesserung der Lagerstabilität werden bekanntermaßen den Dichtungsmassen Stabilisatoren wie Benzoylchlorid, Acetylchlorid, Toluolsulfonsäuremethylester, Carbodiimide und/oder Polycarbodiimide zugesetzt. Als besonders gute Stabilisatoren haben sich Olefine mit 8 bis 20 Kohlenstoffatomen erwiesen. Neben der stabilisierenden Wirkung können diese auch Aufgaben von Weichmachern bzw. Quellmitteln erfüllen. Bevorzugt werden Olefine mit 8 bis 18 Kohlenstoffatomen, insbesondere wenn die Doppelbindung in 1,2-Stellung angeordnet ist. Beste Ergebnisse erhält man, wenn die Molekülstruktur dieser Stabilisatoren linear ist.

Durch die erfindungsgemäße Verwendung von Patchouliöl, Patchoulialkohol und/oder Ester und Ether von Patchoulialkohol, Patchoulenol, Norpatchoulenol und Seychellen zur Verminderung der Anhaftung von Mikrooganismen, insbesondere von Schimmelpilzen an Oberflächen, sowie der asexuellen Vermehrung von Pilzen, insbesondere Schimmelpilzen, umgeht man das Problem der Resistenzbildung aufgrund biozider Wirkstoffe. Bei der Anwendung in schimmelgefährdeten Bau- und Bauhilfsstoffen, insbesondere in Klebstoffen, Anstrichsstoffen und Dichtungsmassen, besonders bevorzugt in Fugendichtungsmassen, werden durch die Verminderung der Anhaftung von Schimmelpilzen an Oberflächen bzw. durch die Hemmung der Sporenbildung mehrere erwünschte Effekte erzielt:
a) Verhinderung von Verfärbungen durch pigmentierte Schimmelpilze .
b) Verzögerung der Ausbreitung des Schimmelbefalls.
c) Verminderung der Allergenbelastung.

Eine weitere bevorzugte Ausführungsform der vorliegenden Erfindung sind Tapetenkleber, enthaltend 0,000001 bis 3 Gew.-% Patchouliöl, Patchoulialkohol und/oder dessen Derivate. Tapetenkleister aus wässrigen Lösungen von Hydrokolloiden wie Methylcellulose, Methylhydroxypropylcellulose oder wasserlöslichen Stärkederivaten. Auch wässrige Dispersionen von filmbildenden Hochmolekularen wie Polyvinylacetat können, insbesondere in Verbindung mit den bereits erwähnten Cellulose- und Stärkederivaten, eingesetzt werden.

Bei der Verwendung in Filtermedien können alle bekannten Arten eingesetzt werden, solange sie für den Einsatz in Wasser- oder Luftfilteranlagen insbesondere für Klimaanlagen oder Raumluftbefeuchter geeignet sind. Insbesondere sind Filtermaterialien aus Cellulose, Glasfasern, PVC-Fasern, Polyesterfasern, Polyamidfasern, insbesondere Nylonfasern, Vliesstoffen, Sintermaterialien und Membranfilter zu nennen.
Die Konzentration des zur Verminderung der Anhaftung von Mikrooganismen an Oberflächen einzusetzenden Patchouliöls, Patchoulialkohols und/oder Ester und Ether von Patchoulialkohol, Patchoulenol, Norpatchoulenol und Seychellen in den erfindungsgemäßen Mitteln kann durch den Fachmann in einem breiten Bereich variiert werden, abhängig von den Einsatzbedingungen der Mittel. Die erfindungsgemäßen Mittel werden nach üblichen und dem Fachmann bekannten Rezepturen hergestellt. Patchouliöl, Patchoulialkohol und/oder Ester und Ether von Patchoulialkohol, Patchoulenol, Norpatchoulenol und Seychellen werden vorzugsweise den bereits fertig zubereiteten Mitteln zugegeben, sie können aber auch während des Herstellungsprozesses zugesetzt werden, wenn dies gewünscht ist.

### Beispiel 1

### Auswirkung von Patchouliöl auf die Sporulation von Aspergillus niger

Kontamination der Oberfläche von Würzeagarplatten mit je 100 µl einer Keimsuspension (10 ³ KBE/ml) von *Aspergillus niger* (DSM1988). Den Agarplatten wurden zuvor unterschiedliche Mengen Wirkstoff (gelöst in Ethanol, Endkonzentrationen siehe Tabelle 1) beigefügt. Die Platten wurden 3 Tage bei 25 °C inkubiert. Die Sporenbildung wurde visuell beurteilt und die Sporulationsrate in [%] bestimmt. Alle eingesetzten Wirkstoffkonzentrationen behinderten das Wachstum des Prüfstamms nicht. Die Sporenbildung wurde mit steigenden Konzentrationen gehemmt und bei 150 µM vollständig unterdrückt.

**Tabelle 1:**

| | | | | | | | | | |
|---|---|---|---|---|---|---|---|---|---|
| **Konzentration Patchouliöl [µM]** | 0 | 3 | 30 | 45 | 60 | 75 | 150 | 225 | 450 |
| **Sporulation [%]** | 100 | 95 | 95 | 95 | 75 | 70 | 0 | 0 | 0 |

**Tabelle 2: Kontrolle**

| | | | | | | |
|---|---|---|---|---|---|---|
| **Konzentration Farnesol [µM]** | 0 | 25 | 62,5 | 125 | 250 | 500 |
| **Sporulation [%]** | 100 | 90 | 75 | 50 | 10 | 0 |

| | | | | | | |
|---|---|---|---|---|---|---|
| Wirkstoff: Patchouliöl, gewonnen mittels Wasserdampfdestillation (Gemisch aus Patchouliöl von Kaders, Sensient, Polarome und Nitsche) | | | | | | |

**Tabelle 3: Zuaammensetzung Patchouliöl**

| **Typanalyse Patchouliöl** | | |
|---|---|---|
| **%** | **Bezeichnung** | **CAS-Nr. 1** |
| 32,0 | Patchoulialkohol | 5986-55-0 |
| 17,1 | Bulnesen | 3691-11-0 |
| 14,3 | Guaien, alpha- | 654-48-6 |
| 8,2 | Seychellen | 20085-93-2 |
| 5,1 | Patchoulen, alpha- | 560-32-7 |
| 3,4 | Caryophyllen | 87-44-5 |
| 2,4 | Pogostol | 21698-41-9 |
| 2,1 | Patchoulen, beta- | 514-51-2 |
| 1,0 | Humulen, alpha- | 6753-98-6 |
| 0,3 | Caryophyllenoxid | 1139-30-6 |
| 0,3 | Copaen | 3856-25-5 |
| 0,2 | Pinen, beta- | 127-91-3 |
| 0,2 | d-Limonen | 5989-27-5 |
| 0,1 | Pinen, alpha- | 80-56-8 |
| 0,1 | Pentadecan | 629-62-9 |
| 13,2 | nicht charakterisierte Komponenten | - |

### Beispiel 2:

### Auswirkung von Patchoulialkohol auf die Sporulation von Aspergillus niger

Kontamination der Oberfläche von Würzeagarplatten mit je 100 µl einer Keimsuspension (10 ³ KBE/ml) von *Aspergillus niger* (DSM1988). Den Agarplatten wurden zuvor unterschiedliche Mengen Wirkstoff (Lösungen in Ethanol, Endkonzentrationen siehe Tabelle 2) beigefügt. Die Platten wurden 5 Tage bei 25 °C inkubiert. Die Sporenbildung wurde visuell beurteilt und die Sporulationsrate in [%] bestimmt. Die eingesetzten Wirkstoffkonzentrationen behinderten das Wachstum des Prüfstamms nicht. Die Sporenbildung wurde mit steigenden Konzentrationen gehemmt und bei 450 µM zu 95% unterdrückt.

**Tabelle 4:**

| | | | |
|---|---|---|---|
| **Konzentration Patchoulialkohol [µM]** | 0 | 225 | 450 |
| **Sporulation [%]** | 100 | 95 | 5 |

### Beispiel 3:

### Auswirkung von Patchouliöl auf die Sporulation von Aspergillus niger auf der Oberfläche einer Acetat-Fugendichtungsmasse

Handelsübliche, jedoch unkonservierte einkomponentige, Raumtemperatur vernetzende Silikon-Fugendichtungsmassen (Acetatsystem, ausgehärtete Filmstücke à 2,2 x 2,2 x 0,3 cm) wurden mit 70% EtOH (Ethanol) desinfiziert und 24h in unterschiedlich konzentrierte Wirkstofflösung eingelegt. Anschließend wurden die Prüfkörper erneut 2 x mit EtOH gewaschen, mit destilliertem Wasser (steril) abgespült und 24h getrocknet. Die Prüfkörper wurden vor und nach dieser Behandlung gewogen und so zusammen mit der Konzentration der Wirkstofflösung die Wirkstoffmenge in den Prüfkörpern bestimmt. Anschließend wurden die Prüfkörper auf Würzeagarplatten aufgelegt und dünn mit Agar, in dem Pilzsporen (10⁵ KBE / ml *Aspergillus niger,* DSM1988) inkorporiert waren, überschichtet. Die Platten wurden 3 Tage bei 25 °C inkubiert. Die Sporenbildung über den Prüfkörpern wurde visuell beurteilt und die Sporulationsrate in [%] bestimmt. Alle getesteten Wirkstoffkonzentrationen behinderten das Wachstum des Prüfstamms nicht. Die Sporenbildung wurde mit steigenden Konzentrationen Patchouliöl gehemmt und bei 9 µM / g Fugendichtungsmasse vollständig unterdrückt.

In einer parallelen Versuchsreihe mit Farnesol als Wirkstoff konnte ebenfalls eine Sporulationshemmung des Teststamms gezeigt werden, die im Vergleich zu gleichen Konzentrationen Patchouliöl deutlich schlechter ist.

**Tabelle 5:**

| | | | | | |
|---|---|---|---|---|---|
| **Konzentration Patchouliöl [µM / g Fugendichtungsmasse]** | 0 | 0,12 | 9 | 33 | 770 |
| **Sporulation [%]** | 100 | 100 | 0 | 0 | 0 |

**Tabelle 6: Kontrolle**

| | | | | |
|---|---|---|---|---|
| **Konzentration Farnesol [µM / g Fugendichtungsmasse]** | 0 | 0,04 | 1,1 | 17 |
| **Sporulation [%]** | 100 | 100 | 100 | 30 |

### Beispiel 4:

### Auswirkung von Patchouliöl auf die Sporulation von Aspergillus niger auf einer Filteroberfläche nach zweimaliger Wirkstoffapplikation

Filterpapiere (2 x 2 cm) wurden desinfiziert und 2x mit je 50 µl unterschiedlich konzentrierte Wirkstofflösung (in Ethanol) im Abstand von 1 h beaufschlagt. Anschließend wurden die Prüfkörper getrocknet. Darauf wurden die Prüfkörper auf Würzeagarplatten aufgelegt und die Oberfläche von Würzeagarplatten mit je 100 µl einer Keimsuspension (10 ³ KBE/ml) von *Aspergillus niger* (DSM1988) kontaminiert. Die Platten wurden 3 Tage bei 25 °C inkubiert. Die Sporenbildung wurde visuell beurteilt und die Sporulationsrate in [%] bestimmt. Alle eingesetzten Wirkstoffkonzentrationen behinderten das Wachstum des Prüfstamms nicht. Die Sporenbildung wurde mit steigenden Konzentrationen gehemmt und bei 90 µM zu 95% unterdrückt.

In einer zweiten, parallelen Versuchsreihe mit Farnesol als Wirkstoff konnte ebenfalls eine Sporulationshemmung des Teststamms gezeigt werden, die im Vergleich zu gleichen Konzentrationen Patchouliöl schlechter ist.

**Tabelle 7:**

| | | | | | | | | |
|---|---|---|---|---|---|---|---|---|
| **Konzentration Patchouliöl [µM]** | 0 | 0,5 | 5 | 25 | 35 | 50 | 70 | 90 |
| **Sporulation [%]** | 100 | 100 | 100 | 100 | 80 | 80 | 10 | 5 |
| **Konzentration Farnesol [µM]** | 0 | 0,5 | 5 | 25 | 35 | 50 | 70 | 90 |
| **Sporulation [%]** | 100 | 100 | 100 | 100 | 60 | 80 | 50 | 40 |

### 5. Flüssigwaschmittel mit Patchouliöl

**Tabelle 8:**

| **Rohstoff** | **Menge in Gewichtsprozent** |
|---|---|
| C₁₂-C₁₈ Fettalkohol + 7 EO (Dehydol LT 7, Cognis) | 15 |
| C₁₂-C₁₄ Fettalkohol C₁₂-C₁₈ Fettalkohol + 7 EO (Dehydol LT 7, Cognis)+ 2 EO-sulfat, Natriumsalz (Texapon N 70, Cognis) | 7 |
| C₈₋₁₈ -Fettsäure geschnitten (Kokosölfettsäure, Edenor K12-18, Cognis) | 8 |
| Natriumcitrat | 1,5 |
| Enzyme | + |
| Farbstoff | + |
| Parfüm | + |
| Patchouliöl (CAS 8014-09-3) | 0,4 |
| Wasser | Ad 100 |

### 6. Vorportioniertes Flüssiawaschmittel in Polyvinylalkohol-Folie mit Patchouli-alkohol

**Tabelle 9:**

| **Rohstoff** | Menge in Gewichtsprozent |
|---|---|
| C₁₂₋₁₄-Fettalkohol+5-EO+4-P O (Marlox MO 154, Sasol) | 25 |
| Dodecylbenzolsulfonat-Isopro pylammoniumsalz (LAS-MIPA, Sasol) | 24,5 |
| C₈₋₁₈ -Fettsäure geschnitten (Kokosölfettsäure, Edenor K12-18, Cognis) | 17,5 |
| Ethanol | 3,5 |
| Natriumcitrat | 0,6 |
| Enzyme | 2,0 |
| Wasser | 6,0 |
| Patchoulialkohol | 0,6 |
| Farbstoff | + |
| Parfum | + |
| Propylenglycol | ad 100 |

| | |
|---|---|
| Das vorportionierte Waschmittel wird mit 50 ml dosiert. | |

### 7. Pulverförmiges Waschmittel mit Patchoulialkohol

**Tabelle 10:**

| Rohstoff | Menge in Gewichtsprozent |
|---|---|
| C₁₀-C₁₃-Alkylbenzolsulfonat | 13,3 |
| C₁₂-C₁₈-Alkylsulfat | 5,5 |
| C₁₂-C₁₈-Alkohol mit 7 EO | 5,3 |
| C₁₂-C₁₈-Alkohol mit 4,5 EO | 0,6 |
| Soil Repellent | 0,7 |
| C₁₆-C₁₈ Fettsäure (Edenor ST1 C₁₆-C₁₈, Cognis) | 0,8 |
| Polyethylenglykol Molekulargewicht= 4000 g/mol | 1,8 |
| Phosphonat | 1,0 |
| Polyacrylate | 2,8 |
| Carboxymethylcellulose | 0,9 |
| Polyvinylpyrrolidon | 0,5 |
| Zeolith (wasserfreie Aktivsubstanz) | 32,1 |
| Natriumcarbonat | 4,5 |
| Natriumtricitrat | 3,6 |
| Citronensäure | 3,7 |
| Natriumhydrogencarbonat | 4,9 |
| Natriumsulfat | 3,8 |
| Entschäumer | + |
| Enzyme | + |
| Farbstoff | + |
| Parfüm | + |
| Patchoulialkohol | 0,4 |
| Wasser / Salze | Ad 100 |

| | |
|---|---|
| Das Waschmittel wird mit 75 g dosiert. | |

Patchoulialkohol kann auch als Bestandteil des Parfüms eingearbeitet. Es ist dann in Konzentrationen von 0,1 - 80 % im Parfümöl enthalten und wird über das in der Waschmittelrezeptur enthaltende Parfümöl in die Waschflotte eingetragen.

### 8. Mundwasser:

**Tabelle 11:**

| | Gew.-% |
|---|---|
| Ethanol (96%) | 65 |
| Polyoxyethylensorbitanmonolaureat (Tween^{®} 20, Uniqema) | 2,0 |
| Aromaöl | 10,0 |
| Propylenglykol | 15,0 |
| Triethanolaminisostearat | 2,0 |
| Natriumsaccharinat | 0,5 |
| Patchouliöl (CAS 8014-09-3) | 0,01 |
| Wasser | Ad 100 |

### 9. Zahncreme

**Tabelle 12:**

| | Gew.-% |
|---|---|
| Dicalciumphosphat | 47,5 |
| Glycerin 86 % DAB | 30 |
| Zahnpastenaromaöl | 1,0 |
| Carboxymethylcellulose, Natriumsalz | 1,2 |
| Natriumlaurylsulfat | 1,0 |
| Saccharinlösung 1% | 0,5 |
| Patchouliöl (CAS 8014-09-3) | 0,02 |
| Wasser | Ad 100 |

### 10. Prothesenreiniqungsmittel, pulverförmig

**Tabelle 13:**

| | Gew.-% |
|---|---|
| Natriumperborat-Monohydrat | 25 |
| Natriumsesquicarbonat | 25 |
| Trinatriumphosphat. Wasserfrei | 40 |
| Natriumlaurylsulfat | 0,2 |
| Kieselsäure | 0,5 |
| Aromastoffe | 0,05 |
| Patchoulialkohol | 0,5 |
| Maltodextrin | 9,3 |

### 11. Gebißhaftmittel:

**Tabelle 14:**

| | Gew.-% |
|---|---|
| Natriumalginat | 10 |
| Paraffinöl perliquidum | 90 |
| Patchoulialkohol | 0,01 |

### 12. bis 15.: Tapetenklebstoffe

**Tabelle 15:**

| **Inhaltsstoffe** | **Menge** |
|---|---|
| Methylhydroxyethylcellulose (300 m • Pas in 2 %iger wässriger Lösung, Methoxylgehalt 26 %) | 500 g |
| PVAcetat-Redispersionspulver | 350 g |
| Kaolin | 60 g |
| Cellulosepulver | 50 g |
| Additionsprodukt von 6 Mol Ethylenoxid an 1 Mol Nonylphenol | 10 g |
| Handelsüblicher Konservierungsstoff (Basis Isothiazolinderivat) | 8 g |
| Patchouliöl | 0,1 g |

**Tabelle 16:**

| **Inhaltsstoffe** | **Menge** |
|---|---|
| Methylhydroxyethylcellulose (5000 m • Pas in 2 % wässrige Lösung, Methoxylgehalt 19 %) | 680 g |
| Carboxylmethylstärke (DS 0,22) | 300 g |
| Additionsprodukt von 4 Mol Ethylenoxid an 1 Mol Fettalkohol | 15g |
| Handelsüblicher Konservierungsstoff (Basis Isothialzolinderivat) | 10g |
| Patchoulialkohol | 0,1 g |

**Tabelle 17:**

| **Inhaltsstoffe** | **Menge** |
|---|---|
| Handelsübliche Polyvinylacetatdisper-sionenen (50 % Feststoffgehalt) | 500 g |
| Wasser | 200 g |
| Methylhydroxyethylcellulose (3000 m • Pas in 2 % wässriger Lösung) | 20 g |
| Handelsübliches Konservierungsmittel | 10g |
| Patchouliöl | 0,1 g |

Die erhaltenen Mischungen werden mit Wasser im Verhältnis 1:20 (2) oder 1:25 (3) oder 1:1 (4) angerührt und zum Aufkleben von handelsüblichen Tapeten auf Wandflächen benutzt.

## Patentansprüche

1. Verwendung von Patchouliöl, Patchoulialkohol und/oder Ester und Ether von Patchoulialkohol, Patchoulenol, Norpatchoulenol und Seychellen, zur Hemmung der asexuellen Vermehrung von humanpathogenen Pilzen zu nicht-medizinischen Zwecken.

2. Verwendung von Patchouliöl, Patchoulialkohol und/oder Ester und Ether von Patchoulialkohol, Patchoulenol, Norpatchoulenol oder Seychellen, zur Verminderung der Anhaftung von Mikroorganismen an Oberflächen, **dadurch gekennzeichnet, dass** die Mikroorganismen ausgewählt sind aus der Gruppe der Bakterien und der humanpathogenen Pilze und wobei es sich bei den Oberflächen um keine Oberflächen am menschlichen oder tierischen Körper handelt.

3. Verwendung nach einem der Ansprüche 1 oder 2, **dadurch gekennzeichnet, dass** die Anhaftung von Mikroorganismen und/oder die asexuelle Vermehrung auf Textilien, Keramiken, Metallen, Filtermedien, Baustoffen, Bauhilfsstoffen, Pelzen, Papier, Fellen, Leder und/oder Kunststoffen vermindert bzw. gehemmt wird.

4. Verwendung nach Anspruch 2, **dadurch gekennzeichnet, dass** die Anhaftung von Mikroorganismen an Oberflächen, die in Kontakt mit dem menschlichen Körper kommen, vermindert wird.

5. Verwendung nach Anspruch 4, **dadurch gekennzeichnet, dass** die Anhaftung von Mikroorganismen auf Wäsche, Prothesen oder Zahnersatz vermindert wird.

6. Verwendung nach einem der Ansprüche 2 bis 5, **dadurch gekennzeichnet, dass** die Pilze ausgewählt sind aus Hefen, Schimmelpilzen und/oder keratinophilen Pilzen.

7. Verwendung nach einem der Ansprüche 1 bis 6, **dadurch gekennzeichnet, dass** die Pilze ausgewählt sind aus den Gattungen Aspergillus, Penicillium, Cladosporium und/oder Mucor, insbesondere Aspergillus.

8. Verwendung nach Anspruch 7, **dadurch gekennzeichnet, dass** die Pilze ausgewählt sind unter allen Spezies der Gattung Aspergillus, ganz besonders bevorzugt unter Aspergillus aculeatus, Aspergillus albus, Aspergillus alliaceus, Aspergillus asperescens, Aspergillus awamori, Aspergillus candidus, Aspergillus carbonarius, Aspergillus carneus, Aspergillus chevalieri, Aspergillus chevalieri var. intermedius, Aspergillus clavatus, Aspergillus ficuum, Aspergillus flavipes, Aspergillus flavus, Aspergillus foetidus, Aspergillus fumigatus, Aspergillus giganteus, Aspergillus humicola, Aspergillus intermedius, Aspergillus japonicus, Aspergillus nidulans, Aspergillus niger, Aspergillus niveus, Aspergillus ochraceus, Aspergillus oryzae, Aspergillus ostianus, Aspergillus parasiticus, Aspergillus parasiticus var. globosus, Aspergillus penicillioides, Aspergillus phoenicis, Aspergillus rugulosus, Aspergillus sclerotiorum, Aspergillus sojae var. gymnosardae, Aspergillus sydowi, Aspergillus tamarii, Aspergillus terreus, Aspergillus terricola, Aspergillus toxicarius, Aspergillus unguis, Aspergillus ustus, Aspergillus versicolor, Aspergillus vitricolae und Aspergillus wentii.

9. Verwendung nach einem der Ansprüche 1 bis 6, **dadurch gekennzeichnet, dass** die Pilze ausgewählt sind aus den Spezies der Candida.

10. Verwendung nach Anspruch 2 bis 6, **dadurch gekennzeichnet, dass** die Anhaftung von Pilzen der Gattungen Malassezia, Trichophyton, Microsporum und/oder Epidermophyton, insbesondere Malassezia und Trichophyton, vermindert wird.

11. Verwendung nach Anspruch 2 oder 3, **dadurch gekennzeichnet, dass** die Bakterien ausgewählt sind aus der Gruppe *Propionibacterium acnes, Stapylococcus aureus,* Streptokokken Gruppe A (beta-hämolysierende S.), *S*. *pyogenes, Corynebacterium* spp. (insbesondere *C*. *tenuis, C. diphtheriae, C. minutissimum*), *Micrococcus spp.,* (insbesondere *M*. *sedentarius*), *Bacillus anthracis, Neisseria meningitidis, N. gonorrhoeae, Pseudomonas aeruginosa, P. pseudomallei, Borrelia burgdorferi, Treponema pallidum, Mycobacterium tuberculosis, Mycobacterium spp., Escherichia coli, Streptococcus* spec., (insbesondere *S*. *gordoni*), *S. mutans*), *Actinomyces* spec., insbesondere A. *naeslundii, Salmonella* spec., *Actinobacteria, insbesondere Brachybacterium* spec., alpha-Proteobacteria, insbesondere *Agrobacterium* spec.,
beta-*Proteobacteria*, insbesondere *Nitrosomonas* spec., *Aquabacterium* spec., *Hydrogenophaga,* gamma-*Proteobacteria,* insbesondere *Stenotrophomonas* spec., *Xanthomonas* spec (campestris), *Neisseria* spec. und *Haemophilus* spec.

12. Verwendung nach einem der Ansprüche 1 bis 11, **dadurch gekennzeichnet, dass** Patchouliöl, Patchoulialkohol und/oder Ester und Ether von Patchoulialkohol, Patchoulenol, Norpatchoulenol oder Seychellen, in den eingesetzten Endkonzentrationen nicht biozid oder biostatisch, insbesondere fungizid oder fungistatisch wirken.

13. Verwendung nach einem der vorhergehenden Ansprüche, **dadurch gekennzeichnet, dass** Patchouliöl, Patchoulialkohol und/oder Ester und Ether von Patchoulialkohol, Patchoulenol, Norpatchoulenol oder Seychellen, in Zubereitungen eingesetzt werden, die ausgewählt sind unter Waschmitteln, Reinigungsmitteln, Nachspülmitteln, Handwaschmitteln Handgeschirrspül-mitteln, Maschinengeschirrspülmitteln und Mitteln zur Ausrüstung von Verpackungen, Filtermedien, Klebstoffen, Baustoffen, Bauhilfsstoffen, Textilien, Pelzen, Papier, Fellen oder Leder.

14. Klebstoff auf Wasserbasis enthaltend Patchouliöl, Patchoulialkohol und/oder Ester und Ether von Patchoulialkohol, Patchoulenol, Norpatchoulenol oder Seychellen.

15. Klebstoff nach Anspruch 14, **dadurch gekennzeichnet, dass** es sich um einen Klebstoff zur Befestigung von Tapeten und ähnlichen Wandbelagsstoffen handelt.

16. Fugendichtungsmasse enthaltend Patchouliöl, Patchoulialkohol und/oder Ester und Ether von Patchoulialkohol, Patchoulenol, Norpatchoulenol oder Seychellen.

## Claims

1. Use of patchouli oil, patchouli alcohol and/or esters and ethers of patchouli alcohol, patchoulenol, norpatchoulenol and seychellen for inhibiting the asexual reproduction of human-pathogenic fungi for non-medical purposes.

2. Use of patchouli oil, patchouli alcohol and/or esters and ethers of patchouli alcohol, patchoulenol, norpatchoulenol or seychellen for reducing the adherence of microorganisms to surfaces, **characterized in that** the microorganisms are selected from the group of bacteria and human-pathogenic fungi, and the surfaces not being surfaces on the human or animal body.

3. The use according to either claim 1 or claim 2, **characterized in that** the adherence of microorganisms to and/or asexual reproduction on textiles, ceramics, metals, filter media, building materials, auxiliary building materials, furs, paper, skins, leather and/or plastic materials is reduced or inhibited.

4. The use according to claim 2, **characterized in that** the adherence of microorganisms to surfaces which come in contact with the human body is reduced.

5. The use according to claim 4, **characterized in that** the adherence of microorganisms to laundry, prostheses or dentures is reduced.

6. The use according to one of claims 2 to 5, **characterized in that** the fungi are selected from yeast, mold fungi and/or keratinophilic fungi.

7. The use according to one of claims 1 to 6, **characterized in that** the fungi are selected from the genera Aspergillus, Penicillium, Cladosporium and/or Mucor, in particular Aspergillus.

8. The use according to claim 7, **characterized in that** the fungi are selected from all species of the genus Aspergillus, particularly preferably from Aspergillus aculeatus, Aspergillus albus, Aspergillus alliaceus, Aspergillus asperescens, Aspergillus awamori, Aspergillus candidus, Aspergillus carbonarius, Aspergillus carneus, Aspergillus chevalieri, Aspergillus chevalieri var. intermedius, Aspergillus clavatus, Aspergillus ficuum, Aspergillus flavipes, Aspergillus flavus, Aspergillus foetidus, Aspergillus fumigatus, Aspergillus giganteus, Aspergillus humicola, Aspergillus intermedius, Aspergillus japonicus, Aspergillus nidulans, Aspergillus niger, Aspergillus niveus, Aspergillus ochraceus, Aspergillus oryzae, Aspergillus ostianus, Aspergillus parasiticus, Aspergillus parasiticus var. globosus, Aspergillus penicillioides, Aspergillus phoenicis, Aspergillus rugulosus, Aspergillus sclerotiorum, Aspergillus sojae var.
gymnosardae, Aspergillus sydowi, Aspergillus tamarii, Aspergillus terreus, Aspergillus terricola, Aspergillus toxicarius, Aspergillus unguis, Aspergillus ustus, Aspergillus versicolor, Aspergillus vitricolae and Aspergillus wentii.

9. The use according to one of claims 1 to 6, **characterized in that** the fungi are selected from the Candida species.

10. The use according to claims 2 to 6, **characterized in that** the adherence of fungi of the genera Malassezia, Trichophyton, Microsporum and/or Epidermophyton, in particular Malassezia and Trichophyton, is reduced.

11. The use according to claim 2 or 3, **characterized in that** the bacteria are selected from the group of *Propionibacterium acnes, Stapylococcus aureus,* group A streptococcus (beta-hemolyzing S.), *S*. *pyogenes, Corynebacterium* spp. (in particular *C*. *tenuis, C. diphtheriae, C. minutissimum), Micrococcus spp.,* (in particular *M. sedentarius*), *Bacillus anthracis, Neisseria meningitidis, N. gonorrhoeae, Pseudomonas aeruginosa, P. pseudomallei, Borrelia burgdorferi, Treponema pallidum, Mycobacterium tuberculosis, Mycobacterium spp., Escherichia coli, Streptococcus* spec., (in particular *S*. *gordoni), S. mutans), Actinomyces* spec., in particular *A*. *naeslundii, Salmonella* spec., *Actinobacteria, in particular Brachybacterium* spec., alpha-*Proteobacteria,* in particular *Agrobacterium* spec., beta-*Proteobacteria,* in particular *Nitrosomonas* spec., *Aquabacterium* spec., *Hydrogenophaga,* gamma-*Proteobacteria,* in particular *Stenotrophomonas* spec., *Xanthomonas* spec (campestris), *Neisseria* spec. and *Haemophilus* spec.

12. The use according to one of claims 1 to 11, **characterized in that** patchouli oil, patchouli alcohol and/or esters and ethers of patchouli alcohol, patchoulenol, norpatchoulenol or seychellen in the final concentrations used do not act in a biocidal or biostatic manner, in particular in a fungicidal or fungistatic manner.

13. The use according to one of the preceding claims, **characterized in that** patchouli oil, patchouli alcohol and/or esters and ethers of patchouli alcohol, patchoulenol, norpatchoulenol or seychellen are used in preparations which are selected from washing agents, cleaning agents, rinsing agents, hand-washing agents, hand-dishwashing detergents, machine-dishwashing detergents and agents for finishing packaging, filter media, adhesives, building materials, auxiliary building materials, textiles, furs, paper, skins or leather.

14. A water-based adhesive containing patchouli oil, patchouli alcohol and/or esters and ethers of patchouli alcohol, patchoulenol, norpatchoulenol or seychellen.

15. The adhesive according to claim 14, **characterized in that** said adhesive is an adhesive for fastening wallpaper and similar wall-covering materials.

16. A joint-sealing compound containing patchouli oil, patchouli alcohol and/or esters and ethers of patchouli alcohol, patchoulenol, norpatchoulenol or seychellen.

## Revendications

1. Utilisation d'huile de patchouli, d'alcool de patchouli et/ou d'esters et d'éthers de patchouli, de patchoulénol, de nor-patchoulénol et de seychellene, pour inhiber la reproduction asexuée des champignons pathogènes pour l'homme à des fins non médicales.

2. Utilisation d'huile de patchouli, d'alcool de patchouli, et/ou d'esters et d'éthers de patchouli, de patchoulénol, de nor-patchoulénol ou de seychellene, pour réduire l'adhérence de microorganismes à des surfaces, **caractérisé en ce que** les microorganismes sont choisis dans le groupe des bactéries et des champignons pathogènes pour l'homme, les surfaces n'étant pas des surfaces du corps humain ou animal.

3. Utilisation selon l'une des revendications 1 ou 2, **caractérisée en ce que** l'adhérence de micro-organismes et/ou la reproduction asexuée sur des textiles, des céramiques, des métaux, des matériaux filtrants, des matériaux de construction, des matériaux de construction auxiliaires, des fourrures, le papier, des peaux, le cuir et/ou des matières plastiques est réduite ou inhibée.

4. Utilisation selon la revendication 2, **caractérisée en ce que** l'adhérence de microorganismes sur des surfaces qui entrent en contact avec le corps humain est réduite.

5. Utilisation selon la revendication 4, **caractérisée en ce que** l'adhérence de micro-organismes sur du linge, des prothèses ou des prothèses dentaires est réduite.

6. Utilisation selon l'une des revendications 2 à 5, **caractérisée en ce que** les champignons sont choisis parmi les levures, les moisissures et/ou les champignons kératinophiles.

7. Utilisation selon l'une des revendications 1 à 6, **caractérisée en ce que** les champignons sont choisis parmi les genres Aspergillus, Pénicillium, Cladosporium et/ou Mucor, en particulier Aspergillus.

8. Utilisation selon la revendication 7, **caractérisée en ce que** les champignons sont choisis parmi toutes les espèces du genre Aspergillus, de manière préférée Aspergillus aculeatus, Aspergillus albus, Aspergillus alliaceus, Aspergillus asperescens, Aspergillus awamori, Aspergillus candidus, Aspergillus carbonarius, Aspergillus carneus, Aspergillus chevalieri, Aspergillus chevalieri var. intermedius, Aspergillus clavatus, Aspergillus ficuum, Aspergillus flavipes, Aspergillus flavus, Aspergillus foetidus. Aspergillus fumigatus, Aspergillus giganteus, Aspergillus humicola, Aspergillus intermedius, Aspergillus japonicus, Aspergillus nidulans, Aspergillus niger, Aspergillus Niveus, Aspergillus ochraceus, Aspergillus oryzae, Aspergillus ostianus, Aspergillus parasiticus, Aspergillus parasiticus var. globusus, penicillioides, Aspergillus phoenicis, Aspergillus rugulosus, Aspergillus sclerotiorum, Aspergillus sojae var. gymnosardae, Aspergillus sydowi, Aspergillus tamarii, Aspergillus terreus, Aspergillus terricola, Aspergillus toxicanus, Aspergillus unguis, Aspergillus ustus, Aspergillus versicolor, Aspergillus vitricolae et Aspergillus wentii.

9. Utilisation selon l'une des revendications 1 à 6, **caractérisée en ce que** les champignons sont choisis parmi les espèces de Candida.

10. Utilisation selon l'une des revendications 2 à 6, **caractérisé en ce que** l'adhérence des champignons des genres Malassezia, Trichophyton, Microsporum et/ou Epidermophyton, en particulier Malassezia et Trichophyton est diminuée.

11. Utilisation selon la revendication 2 ou 3, **caractérisée en ce que** les bactéries sont choisies dans le groupe *Propionibacterium acnes, Stapylococcus aureus,* Streptococcus groupe A (bêta-hémolytique de S.), *S*. *pyogenes, Corynebacterium* spp. (en particulier *C*. *tenuis, C. diphtheriae. C. minutissimum*), *Micrococcus* spp., (en particulier *M*. *sedentarius*), *Bacillus anthracis, Neisseria meningitidis, N. gonorrhoeae, Pseudomonas aeruginosa, P. pseudomallei, Borrelia burgdorferi, Treponema pallidum, Mycobaclehum tuberculosis, Mycobacterium spp., Escherichia coli, Streptococcus* spec. (en particulier *S*. *gordoni*), *S. mutans, Actinomyces* spec., en particulier *A. naeslundii*, *Salmonella* spec, *Actinobacteria* spec., en particulier *Brachybacterium* spec., *alpha-Proteobacteria,* notamment *Agrobacterium* spec., beta-Proteobacteria, notamment *Nitrosomonas* spec., *Aquabacterium* spec, *Hydrogenophaga,* gamma-*Proteobacteria,* notamment *Stenotrophomonas* spec., *Xanthomonas* spec. (campestris), *Neisseria* spec. et *Haemophilus* spec.

12. Utilisation selon l'une quelconque des revendications 1 à 11, **caractérisée en ce que** l'huile de patchouli, l'alcool de patchouli et/ou les esters et éthers d'alcool de patchouli, le patchoulénol, le nor-patchoulénol ou le seychellene, agissent aux concentrations finales utilisées de façon non-biocide ou biostatique, en particulier fongicide ou fongistatique.

13. Utilisation selon l'une des revendications précédentes, **caractérisée en ce que** l'huile de patchouli, l'alcool de patchouli et/ou les esters et éthers de patchouli, le patchoulénol, ou le nor-patchoulénol ou le seychellene, sont utilisés dans des préparations qui sont choisies parmi les agents de lavage, les agents de nettoyage, les agents de rinçage, les agents de lavage pour les mains, les agents de lavage de la vaisselle à la main, les agents de lavage de la vaisselle en machine et les agents de finition des emballages, les milieux filtrants, les adhésifs, les matériaux de construction, les matériaux de construction auxiliaires, les textiles, les fourrures, le papier, les peaux ou le cuir.

14. Adhésif à base d'eau contenant de l'huile de patchouli, de l'alcool de patchouli et/ou des esters et éthers d'alcool de patchouli, du patchoulénol, du nor-patchoulénol ou de seychellene.

15. Adhésif selon la revendication 14, **caractérisé en ce qu'**il s'agit d'un adhésif destiné à la fixation de papiers peints et de revêtements muraux similaires.

16. Composé de jointement contenant de l'huile de patchouli, de alcool de patchouli et/ou des esters et éthers d'alcool de patchouli, du patchoulénol, du nor-patchoulénol ou de seychellene.
